# EUROPÄISCHE PATENTSCHRIFT

(11) **EP 4 009 895 B1**
(45) Veröffentlichungstag und Bekanntmachung des Hinweises auf die Patenterteilung: **30.09.2026**
(21) Anmeldenummer: 20753714.3
(22) Anmeldetag: 05.08.2020
(51) Int. Cl.: A61B 18/14, A61B 5/00, G01N 21/359, A61B 90/30, A61B 18/04

(54) **VORRICHTUNG ZUR GEWEBEERKENNUNG**
DEVICE FOR TISSUE IDENTIFICATION
DISPOSITIF D'IDENTIFICATION DE TISSU

(30) Priorität: 07.08.2019 DE 102019121365
(43) Veröffentlichungstag der Anmeldung: 15.06.2022
(73) Patentinhaber: Aesculap AG, 78532 Tuttlingen (DE)
(72) Erfinder: HUBER, Christian, 78570 Mühlheim (DE); WEISSHAUPT, Dieter, 78194 Immendingen (DE); ROTHWEILER, Christoph, 78166 Donaueschingen (DE); RUSS, Detlef, 71272 Renningen (DE); FUGGER, Oliver, 89081 Ulm (DE); HIBST, Raimund, 89155 Erbach (DE); KIENLE, Alwin, 89134 Blaustein (DE); FOSCHUM, Florian, 89233 Neu-Ulm (DE)
(74) Vertreter: Winter, Brandl - Partnerschaft mbB
(86) Internationale Anmeldenummer: PCT/EP2020/072028
(87) Internationale Veröffentlichungsnummer: WO 2021/023781

(56) Entgegenhaltungen:
- EP-A1- 0 694 291
- WO-A1-2009/005850
- CN-A- 110 074 856
- US-A- 5 743 903
- US-A1- 2002 169 445
- US-A1- 2006 111 622
- US-A1- 2012 296 238
- US-A1- 2013 204 134
- US-A1- 2016 346 034
- US-A1- 2017 156 797
- US-A1- 2019 231 193
- RAMI NACHABÉ ET AL: "Estimation of lipid and water concentrations in scattering media with diffuse optical spectroscopy from 900?to?1600?nm", JOURNAL OF BIOMEDICAL OPTICS, vol. 15, no. 3, 1 January 2010 (2010-01-01), pages 037015, XP055329702, ISSN: 1083-3668, DOI: 10.1117/1.3454392
- JEFFREY J. KELLY ET AL: "Tissue temperature by near-infrared spectroscopy", PROCEEDINGS OF SPIE, vol. 2389, 30 May 1995 (1995-05-30), US, pages 818 - 828, XP055744396, ISSN: 0277-786X, DOI: 10.1117/12.210025

## Beschreibung

### Technisches Gebiet

Die Erfindung betrifft ein medizinisches Hochfrequenz-Chirurgie Instrument (HF-, Ultraschall-, Laser-Instrument, etc.) zur Gewebeerkennung insbesondere von menschlichem Gewebe und eine Anwendung eines Gewebeerkennungsverfahrens bei einem medizinischen Hochfrequenz-Chirurgie Instrument (HF-, Ultraschall-, Laser-Instrument etc.) vorzugsweise gemäß der vorliegenden Erfindung.

### Hintergrund der Erfindung

Bei der Hochfrequenz-Chirurgie (im Weiteren als HF-Chirurgie bezeichnet) wird hochfrequenter Wechselstrom durch den menschlichen Körper oder ein Körperteil geleitet, um Gewebe durch die damit verursachte Erwärmung gezielt zu veröden (Koagulation) bzw. zu schneiden (Elektrotomie). Das so geschädigte Gewebe wird später vom umgebenden gesunden Gewebe resorbiert. Ein wesentlicher Vorteil gegenüber herkömmlicher Schneidetechnik mit dem Skalpell ist, dass gleichzeitig mit dem Schnitt eine Blutungsstillung durch Verschluss der betroffenen Gefäße erfolgen kann, im Sinne einer Koagulation. Für das sichere Verschließen von Gefäßen sollten sogenannte Seal&Cut Instrumentarien verwendet werden. Die benutzten Geräte werden auch als Elektroskalpell bezeichnet.

Bei den für die HF-Chirurgie (Hochfrequenz-Chirurgie) verwendeten Frequenzen verhält sich das Körpergewebe wie ein Ohm'scher Widerstand (Impedanz). Der spezifische Widerstand hängt stark von der Gewebeart ab. Der spezifische Widerstand von Muskelgewebe und stark durchblutetem Gewebe ist relativ gering. Der von Fett ist ca. um den Faktor 15 höher und der von Knochen um den Faktor 1000. Frequenz, Form und Höhe des Stroms müssen/sollten somit auf die Gewebeart, an der operiert wird, abgestimmt sein.

Derzeit wird am häufigsten die monopolare HF-Technik in der HF-Chirurgie angewendet. Dabei wird ein Pol der HF-Spannungsquelle über eine möglichst großflächige Gegenelektrode mit dem Patienten verbunden, z.B. durch Kontakte auf dem Operationstisch auf dem der Patient liegt, durch Kontaktarmbänder bzw. Kontaktfußbänder oder durch Klebeelektroden. Diese Gegenelektrode nennt man oft neutrale Elektrode bzw. Neutralelektrode. Der andere Pol wird an das chirurgische Instrument angeschlossen und dieses bildet die sogenannte aktive Elektrode bzw. Aktivelektrode. Der Strom fließt über den Weg des geringsten Widerstandes von der Aktivelektrode zur Neutralelektrode. In unmittelbarer Nähe der Aktivelektrode ist die Stromdichte am höchsten, hier findet der thermische Effekt am stärksten statt. Die Stromdichte nimmt mit dem Quadrat des Abstands ab. Die Neutrale Elektrode sollte möglichst großflächig und mit dem Körper gut verbunden sein, sodass die Stromdichte im Körper gering gehalten wird und keine Verbrennungen stattfinden. Die Haut an der Neutralelektrode wird durch die große Fläche nicht spürbar erwärmt. Bei der Anbringung der Neutralelektrode gelten strenge Sicherheitsmaßnahmen. Um keine Verbrennungen zu verursachen, sind richtige Position und guter Kontakt der neutralen Elektrode (abhängig vom Operationsgebiet) ausschlaggebend.

Bei der bipolaren HF-Technik fließt der Strom im Gegensatz zur monopolaren Technik durch einen kleinen Teil des Körpers - denjenigen, in dem die chirurgische Wirkung (Schnitt oder Koagulation) gewünscht ist. Zwei gegeneinander isolierte Elektroden (z.B. in Instrumentenbranchen aufgenommen), zwischen denen die HF-Spannung anliegt, werden direkt an die Operationsstelle geführt. Der Stromkreis wird über das dazwischen liegende Gewebe geschlossen. In dem Gewebe zwischen den Elektroden findet der thermische Effekt statt.

Koagulationsklemmen sind bekannt. Die Hochfrequenzanschlüsse sind dabei in der Regel an den Handgriffen/ dem Handgriff vorgesehen. Als Achse für das Gelenk dient häufig eine mit einem Isolierüberzug versehene Schraube, mit welcher auch die beiden Klemmschenkel mit ihren Handgriffen jeweils schwenkbar aneinander befestigt sind.

Mit einem bipolaren HF-Gefäßversiegelungs- und/oder Schneidsystem können Gefäße oder Gewebebündel allgemein oder während des Schneidens effektiv und dauerhaft versiegelt werden. Somit wird die laterale thermische Schädigung des umliegenden Gewebes begrenzt, und Gewebeanhaftungen werden auf ein Minimum reduziert.

Als Gewebe bezeichnet man in der Medizin ein organisches Material, das aus einer Gruppe gleichartig oder unterschiedlich differenzierter Zellen besteht, die eine gemeinsame Funktion oder Struktur aufweist. Zum Gewebe gehört neben den Zellen auch die extrazelluläre Matrix (EZM). Beispiele für menschliche Gewebe sind z.B. Blutgefäße.

Der menschliche Körper besteht in seiner chemischen Zusammensetzung aus ca. 56% Sauerstoff (O), 28% Kohlenstoff (C), 9% Wasserstoff (H), 2% Stickstoff (N), 1.5 % Calcium, 1% Chlor (Cl), 1% Phosphor (P), 0,25% Kalium (K), 0,2% Schwefel (S) und andere chemischen Substanzen in kleineren Anteilen (alle Angaben in Gewichtsprozent).

Die Substanzzusammensetzung des menschlichen Körpers besteht aus ca. 67% Wasser, 16% Proteine bzw. Eiweiß (z.B. Kollagene), 10% Lipide (z.B. Fett), 1% Kohlenhydrate, 1% Nucleinsäuren und 5% diverser Mineralstoffe (alle Angaben in Gewichtsprozent).

Kollagene sind eine Gruppe von bei Menschen und Tieren vorkommender Strukturproteine (ein Faserbündel bildendes "Eiweiß") hauptsächlich des Bindegewebes (genauer: der extrazellulären Matrix). Kollagene finden sich unter anderem in den weißen, unelastischen Fasern von Sehnen, Bändern, Knochen und Knorpeln. Auch Schichten der Haut (Unterhaut) bestehen aus Kollagenen. Im menschlichen Körper ist Kollagen mit über 30 % Anteil an der Gesamtmasse aller Proteine das am häufigsten vorkommende Eiweiß.

In lebenden Organismen werden Lipide hauptsächlich als Strukturkomponenten in Zellmembranen, als Energiespeicher oder als Signalmoleküle gebraucht. Oft wird der Begriff "Fett" als Synonym für Lipide gebraucht, jedoch stellen die Fette (Triglyceride) nur eine Untergruppe der Lipide dar.

Optische Hauptabsorber in Gewebe wie zum Beispiel in Blutgefäßen im NIR-Bereich sind Wasser und Kollagen. Die Blutgefäße sind meist von Fett umgeben.

Bei Wechselwirkung elektromagnetischer Strahlung mit Festkörpern, Flüssigkeiten oder Gasen treten verschiedene Effekte wie Absorption, Reflexion, Streuung oder Transmission auf. In anderen Worten, trifft die elektromagnetische Strahlung auf ein Hindernis, wird sie entweder absorbiert (verschluckt), gestreut (aus ihrer ursprünglichen Richtung abgelenkt), transmittiert (hindurchgelassen) oder reflektiert (zurückgeworfen) - man spricht auch von Remission bei der Reflexion.

Als Remission bezeichnet man in der Physik die diffuse (ungerichtete) elektromagnetische Strahlung, insbesondere von Licht, die durch die Oberfläche in ein streuendes Medium eindringt, mit diesem wechselwirkt und durch diese Oberfläche wieder austritt. Im Gegensatz zur regulären gerichteten Reflexion, die das Reflexionsgesetz erfüllt. Häufiger wird aber in beiden Fällen von Reflexion gesprochen. Man unterscheidet dann zwischen spiegelnder und diffuser Reflexion. Bei der Remission (diffusen Reflexion) wird ein Teil des Lichts absorbiert und transmittiert. Das oberflächenbezogene Maß für die Remission ist der Remissionsgrad.

Die Remissionsspektroskopie ist ein Teilgebiet der Spektroskopie, das die von einer Probe remittierte Strahlung misst. Die Remissionsspektroskopie dient vornehmlich der spektralen Untersuchung lichtundurchlässiger und unlöslicher Proben. Das gemessene Remissionsspektrum einer Probe besteht aus zwei Anteilen: 1) der regulären Reflexion, bei der die Strahlung spiegelnd von der Oberfläche reflektiert wird. Sie wird durch die Fresnelschen Gleichungen beschrieben; 2) der diffusen Remission, bei der die Strahlung isotrop in alle Richtungen aus der Probe austritt. Sie kommt dadurch zustande, dass die Strahlung in die Probe eindringt und nach teilweiser Absorption und mehrfacher Streuung an die Oberfläche zurückgelangt.

Das jeweilige Absorptionsspektrum von Wasser, Kollagen und Fett ist von zahlreichen Gruppen bereits vermessen worden. Sowohl im sichtbaren Spektralbereich (VIS) als auch im nahen Infrarotspektralbereich (NIR) sind die Werte für die Absorptionskoeffizienten verfügbar.

Die Steuerung der Regelprozesse bei der bipolaren HF-Technik findet im Stand der Technik über die Gewebeimpedanz statt, welche sich im Verlauf der Energiezufuhr, vorwiegend durch den Verlust von Wasser, ändert. Die Impedanz des Gewebes wird mittels des Ohm'schen Gesetzes anhand der gemessenen Spannungs- und Stromwerte berechnet. Aufgrund der Konfiguration eines Instruments ist die ermittelte Impedanz immer ein Durchschnittswert des gesamten Systems (Gewebe, Instrument, Kabel, Generator).

Die Qualität der Versiegelung von Blutgefäßen hängt im Wesentlichen von dem Regelprozess und dem damit verbundenen Energieeintrag in das Gewebe ab. Hierbei kann es neben der Überhitzung der Instrumente auch zur thermischen Schädigung des umliegenden Gewebes kommen. Ebenso kann ein nicht ausreichender Energieeintrag auch zum Versagen/Aufplatzen der fusionierten Stellen führen, was sich wiederum durch Blutungen bemerkbar macht. Häufig treten dies Blutungen erst Stunden nach der eigentlichen Operation auf, so dass, je nach Gefäßdurchmesser, Notoperationen erforderlich werden können, um die Blutung zu stoppen, bzw. das Gefäß sicher zu verschließen.

### Stand der Technik

Aus dem Stand der Technik ist es daher bekannt, die Gewebetemperatur zu messen und die gemessenen Temperaturwerte in die Regelung/ Steuerung der thermischen Prozesses einfließen zu lassen. Um die Temperatur-Messergebnisse nicht durch die Elektrodentemperatur zu verfälschen, ist ein ausreichend großer Abstand, bzw. eine thermische Trennung/Isolation zwischen Gewebetemperatursensor und Elektrode(n) erforderlich. Dies ist jedoch insofern nachteilig, als dass die gemessene Gewebetemperatur nicht exakt jener Gewebetemperatur unmittelbar an der (den) Elektrode(n) entspricht.

Die US 20170156797 A1 beschreibt eine Methode zur Bestimmung eines Gewebeversiegelungszustands während eines Gewebeversiegelungsprozesses.

Die US 2013/204134 A1 beschreibt eine Eigenschaftsbestimmungsvorrichtung zur Bestimmung einer Eigenschaft eines Objekts.

Die US 2016/346034 A1 beschreibt ein medizinisches Instrument mit einem Gehäuse und einem mit dem Gehäuse gekoppelten Schaft.

Die US 5743903 beschreibt ein Herzablationssystem und -verfahren unter Verwendung einer Ablationselektrode mit einem energieemittierenden Körper.

Die US 2002/169445 A1 stellt einem Prozessor Informationen bereit, die die Fließgeschwindigkeit eines Fluids durch ein biologisches Organ anzeigen.

Die CN 110074856 A beschreibt ein Steuerverfahren, eine Steuervorrichtung und ein computerlesbares Speichermedium für Hochfrequenzablation, einen Hochfrequenzablationskatheter und ein Lungen-Hochfrequenzablationssystem.

XP 55329702 A beschreibt eine Schätzung von Lipid- und Wasserkonzentrationen in streuenden Medien mit diffuser optischer Spektroskopie von 900 bis 1600 nm.

XP 55744396 A betrifft eine Gewebetemperatur durch Nahinfrarotspektroskopie.

Die WO 2009/005850 A1 offenbart ein Instrument, das so konfiguriert ist, dass es die Wasserkonzentration anhand optischer Informationen erkennt und die Energiezufuhr auf der Grundlage dieser Informationen steuert. Die US 2012/296238 A1 beschreibt ein System, das Lichtinformationen als Rückkopplung für die Steuerung der Energieabgabe verwendet. Weiterer Stand der Technik ist aus der US 2019/231193 A1, der US 2006/111622 A1 und der EP 0 694 291 A1 bekannt.

### Kurzbeschreibung der Erfindung

Die Aufgabe der Erfindung ist es deshalb, zusätzlich oder alternativ zu der Messung der Impedanz eine möglichst exakte Messung des Gewebes/ der Gewebezusammensetzung und/oder der Temperatur des zu fusionierenden Gewebes zu ermöglichen, vorzugsweise online, um eine Schädigung des Gewebes unmittelbar an der (den) Elektrode(n) zu vermeiden und ggf. auch eine Überhitzung der Instrumente zu verhindern. In anderen Worten ausgedrückt ist es die Aufgabe der Erfindung eine gute Koagulation zu ermöglichen.

Die Aufgabe der Erfindung wird gelöst durch die Merkmale des Anspruchs 1.

Die Erfindung betrifft ein medizinisches Hochfrequenz-Chirurgie Instrument mit
- zumindest einer Instrumentenbranche,
- zumindest eine Lichtquelle (z.B. LED) oder Lichtquellenbaugruppe (z.B. LED und Filter), die ein erstes Licht mit einem bestimmten Beleuchtungs-Lichtspektrum erzeugt, welches in Richtung hin zu einem Gewebe (direkt oder indirekt) aussendbar ist, und
- zumindest einem Sensor, der dafür vorgesehen und angepasst ist, ein zweites Licht mit einem (vom Beleuchtungs-Lichtspektrum ggf. unterschiedlichen) Remissionsspektrum zu erfassen, welches von dem Gewebe infolge der Lichtbeaufschlagung durch die Lichtquelle reflektiert wird, und
- das zweite Licht entsprechend dessen Remissionsspektrums in ein Detektorsignal umzuwandeln, wobei
   eine Recheneinheit dazu vorgesehen und ausgebildet ist, um
- das Detektorsignal von dem zumindest einen Sensor zu empfangen,
- ein theoretisches Remissionsspektrum basierend auf einer Lösung zur Beschreibung der Lichtausstrahlung im Gewebe, vorzugsweise basierend auf der Strahlungstransporttheorie und ihrer Näherungen, mittels der Recheneinheit (1144), unter Annahme geschätzter Volumenanteile der einzelnen Gewebebestandteile, die im Gewebe vorhanden sind, zu berechnen,
- das theoretische Remissionsspektrum an das gemessene Remissionsspektrum beispielsweise durch eine nichtlineare Regression, ein Neuronales Netz oder eine Look-Up-Tabelle mittels der Recheneinheit anzupassen, und
- wenigstens einen Volumenanteil eines Gewebebestandteils aus dem Remissionsspektrum über einen Minimierungsalgorithmus zu berechnen, mit dem das Theoretisch berechnete Remissionsspektrum an das gemessene Remissionsspektrum mittels der Recheneinheit durch Variation der Volumenanteile der einzelnen Gewebebestandteile, die im Gewebe vorhanden sind, angefittet (angepasst) wird,
- wenigstens ein Absorptionsmaximum aus einem Absorptionsspektrum des Gewebes zu berechnen, wobei das Absorptionsspektrum des Gewebes aus dem Fit des theoretisch berechneten Remissionsspektrums an das gemessene Remissionsspektrum ermittelt wird,
- eine Temperatur in dem Gewebe durch Vergleich des Absorptionsmaximums mit wenigstens einer Referenz zu berechnen,
- das medizinische Hochfrequenz-Chirurgie Instrument auf Basis des berechneten Volumenanteils eines Gewebebestandteils und der berechneten Temperatur, oder auf Basis des berechneten Volumenanteils eines Gewebebestandteils, der berechneten Temperatur und berechneter Gewebeimpedanz zu steuern und/oder zu regeln und/oder abzuschalten.

Die Erfindung ist im beigefügten Anspruchssatz beschrieben.

Eine Verwendung eines Gewebeerkennungsverfahrens zur Steuerung des medizinischen Hochfrequenz-Chirurgie-Instruments mit zumindest einer Instrumentenbranche, insbesondere eines Seal&Cut Instruments, besonders bevorzugt während eines thermischen Verfahrens/ Prozesses, mit den folgenden Schritten (vorzugsweise in dieser Reihenfolge):
- Erzeugen eines ersten Lichts, mit einem Beleuchtungsspektrum/ Anregungsspektrum, vorzugsweise im VIS-/NIR-Bereich, welches in Richtung hin zu einem Gewebe mittels wenigstens einer Lichtquelle/ Beleuchtung aussendbar ist,
- Empfangen der Remission des eingestrahlten Lichtes, mit einem Remissionsspektrum, aus dem Gewebe durch wenigstens einen Detektor, vorzugsweise einem Sensor, bzw. in anderen Worten, messen eines zweiten Lichts, das durch Remission des eingestrahlten ersten Lichts erhalten/empfangen wird, mit einem Remissionsspektrum, aus dem Gewebe durch zumindest einen Sensor/ Detektor,
- Umwandeln des gemessenen Remissionsspektrums mittels des zumindest einen Sensors/ Detektors in ein Detektorsignal, vorzugsweise ein elektrisches Signal/Datensignal,
- Senden des Detektorsignals an eine Recheneinheit, vorzugsweise eine CPU,
- Berechnen eines theoretischen Remissionsspektrums basierend auf einer Lösung/ eines Algorithmus zur Beschreibung der Lichtausbreitung im Gewebe, vorzugsweise basierend auf der Strahlungstransporttheorie und ihrer Näherungen, mittels der Recheneinheit, bevorzugt unter Annahme geschätzter Volumenanteile der einzelnen Gewebebestandteile, die im Gewebe vorhanden sind,
- Anpassung/ Fit des theoretischen Remissionsspektrums an das gemessene Remissionsspektrum, vorzugsweise durch eine Regression, ein Neuronales Netz oder eine Look-Up-Tabelle, mittels der Recheneinheit, bzw. in anderen Worten berechnen wenigstens eines Volumenanteils eines Gewebebestandteils durch Variation der Volumenanteile der einzelnen Gewebebestandteile, die im Gewebe vorhanden sind, über einen Minimierungsalgorithmus, mit dem das berechnete theoretischen Remissionsspektrum an das gemessene Remissionsspektrum vorzugsweise durch eine Regression, ein Neuronales Netz oder eine Look-Up-Tabelle mittels der Recheneinheit angefittet oder angepasst wird, und
- Berechnen wenigstens eines Volumenanteils eines Gewebebestandteils durch Variation der Volumenanteile der einzelnen Gewebebestandteile, die im Gewebe vorhanden sind, über einen Minimierungsalgorithmus, mit dem das theoretisch berechnete Remissionsspektrum an das gemessene Remissionsspektrum, vorzugsweise durch eine Regression, ein Neuronales Netz oder eine Look-Up-Tabelle mittels der Recheneinheit angefittet (angepasst) wird.

Ein thermischer Prozess ist vorzugsweise jeder Prozess, der durch Energieabgabe thermische Effekte im Gewebe erzeugt. Darunter zählen auch Prozesse die mittels Hochfrequenz, Ultraschall, Laser und/ oder Temperatur erfolgen. Auch zählen Prozesse darunter, die mittels Hochfrequenz-, Ultraschall-, Laser- und/ oder Temperaturinstrumenten erfolgen (z.B. mittels Thermokauter), beziehungsweise alle medizinischen Instrumente, die durch Energieabgabe thermische Effekte im Gewebe erzeugen. Unter Beleuchtungsspektrum oder Anregungsspektrum ist vorzugsweise das Spektrum (bzw. der Wellenlängenbereich) der Lichtquelle zu verstehen.

Der Kern der vorliegenden Erfindung besteht demzufolge darin, Gewebebestandteileüber die Anpassung/ den Fit von berechneten Remissionsspektren an das gemessene Remissionsspektrum bei Variation von wenigstens einem Parameter, falls das Streuspektrum des Gewebes als bekannt vorausgesetzt wird, oder bei Variation von mindestens zwei Parametern, wovon mindestens einer einen unmittelbaren oder mittelbaren Rückschluss auf die Streuung von Gewebe ermöglicht und die anderen Parameter einen unmittelbaren oder mittelbaren Rückschluss auf die Bestandteile, vorzugsweise deren Volumenanteil, von Gewebe ermöglicht (kausaler Zusammenhang zwischen den Gewebebestandteilen und dem Parameter), zu bestimmen. Bevorzugte Gewebebestandteile sind Wasser, Fett und/oder Kollagen. Aus den aus dem Fit erhaltenen Parametern kann ermittelt werden welche Gewebebestandteile mit welchem Volumenanteil in dem Gewebe vorhanden sind, da das für den Fit berechnete Absorptionsspektrum des Gewebes über die im Gewebe bekannten charakteristischen Bestandteile kombiniert wird. In anderen Worten ausgedrückt ist bekannt, dass in dem Gewebe Wasser, Kollagen und Fett vorhanden ist, so kann mit Hilfe von deren charakteristischen Absorptionsspektren, das gemessene Absorptionsspektrum als Überlagerung der einzelnen charakteristischen Absorptionsspektren der einzelnen Gewebebestandteile kombiniert werden. Somit kann mittels eines Fitalgorithmus berechnet werden, in welchem Volumenanteil die einzelnen Gewebebestandteile vorliegen.

In wieder anderen Worten ausgedrückt, kann mittels der Recheneinheit ermittelt werden in welchem Volumenanteil die einzelnen Gewebebestandteile in dem Gewebe vorhanden sind. Das bedeutet, dass aus dem Fit der theoretisch berechneten Remissionsspektren an das gemessene Remissionsspektrum (das von der Recheneinheit aus dem Signal von dem Detektor berechnet wurde) das Absorptionsspektrum des Gewebes unabhängig von den Streueigenschaften des Gewebes ermittelt wird. Die Absorptionsspektren der einzelnen Gewebebestandteile, vorzugsweise von Wasser, Fett und Kollagen, sind auf einem Speichermedium in der Recheneinheit gespeichert. Somit können die Volumenanteile der Gewebebestandteile aus einem von dem Detektor gemessenen Remissionsspektrum, von der Recheneinheit berechneten Absorptionsspektrum über eine Linearkombination der Absorptionsspektren der einzelnen Gewebebestandteile mit den Volumenanteilen als Vorfaktoren berechnet werden berechnet werden, wodurch die CPU die Gewebezusammensetzung, also den prozentualen Volumenanteil, der einzelnen Bestandteile ermitteln kann.

Vorzugsweise ist mittels der Recheneinheit, aufgrund der berechneten Gewebebestandteile und/oder der berechneten Temperatur und/oder der berechneten Gewebeimpedanz, der Strom, die Spannung und/oder die Frequenz der Elektroden, in Erwiderung auf die ermittelte Gewebezusammensetzung und/oder der berechneten Temperatur (auf Basis Detektorsignals) und/oder der berechneten Gewebeimpedanz, von der Recheneinheit steuerbar und/oder regelbar sein.

Vorzugsweise weist das Gewebeerkennungsverfahren ferner den Schritt auf: Berechnen wenigstens eines Absorptionsmaximums aus dem Absorptionsspektrum mittels der Recheneinheit, berechnen einer Temperatur in dem Gewebe durch Vergleich des Absorptionsmaximums mit wenigstens einer Referenz mittels der Recheneinheit, speichern wenigstens einer Referenz in Form eines Absorptionsmaximums bei einer bestimmten Temperatur in der Recheneinheit, vorzugsweise einem Speichermedium in der Recheneinheit, vorzugsweise für Wasser und/oder Fett und/oder Kollagen.

Vorzugsweise ist die Gewebezusammensetzung/ sind die Gewebebestandteile eine wichtige Prädiktionsgröße für die Güte eines medizinischen Instruments und/oder eines Koagulationsverfahrens und/oder eines HF-Sealings. Insbesondere der Kollagenbestandteil ist ein wichtiger Parameter. Vorzugsweise ist es von besonderer Bedeutung abhängig von den Gewebebestandteilen die Prozessführung anzupassen. Zur Bestimmung der Gewebebestandteile werden vorzugsweise, wie bei einer Temperaturmessung, die Spektren/ das Spektrum der Remission aufgenommen. Das heißt, breitbandige Strahlung im sichtbaren und infraroten Spektralbereich wird über eine Beleuchtung in das Gewebe eingestrahlt und die aus dem Gewebe zurückgestreute/remittierte Strahlung spektral detektiert, vorzugsweise in einem Abstand zum Einstrahlungsort. Das so gemessene Remissionsspektrum ist abhängig von den Streu- und Absorptionseigenschaften der verschiedenen Gewebekonstituenten/ Gebebestandteile. Durch einen Algorithmus /Rechenverfahren , der/ das die Streueigenschaften und die Absorptionseigenschaften berücksichtigt, können die Volumenanteile der absorbierenden Gewebebestandteile so angepasst werden, dass durch die Überlagerung der einzelnen Absorptionsspektren der reinen Gewebebestandteile ein Absorptionsspektrum erzeugt wird, mit dem theoretisch in Verbindung mit geeigneten Streueigenschaften ein Remissionsspektrum berechnet werden kann, das mit dem gemessenen Remissionsspektrum übereinstimmt. Auf diese Weise kann der Wasser-, Fett- und Kollagengehalt des nativen Gewebes/ des gemessenen Gewebes vor und/oder während des Verfahrens/ des Sealingprozesses bestimmt werden. Bei Verwendung von temperaturabhängigen Spektren kann die Veränderung der Gewebezusammensetzung, etwa durch Verdampfen von Wasser, auch während des Prozesses verfolgt werden. Auf diese Weise kann die Abnahme von Wasser/ des Wassergehalts im Gewebe während des Sealings erfasst und als Regel-/Steuer- und/oder Abschaltparameter angewendet werden. Auch die Unterscheidung verschiedener Gewebetypen könnte auf diese Weise möglich sein.

Die Gewebeanteile des Gewebes weisen eine typische Absorptionskennlinie auf. So hat Wasser beispielsweise ein Absorptionsmaximum bei ca. 1470nm bei Raumtemperatur, Kollagen dagegen hat ein Absorptionsmaximum bei ca. 1500nm bei Raumtemperatur und Fett hat je ein Absorptionsmaximum bei 1210nm und bei ca. 1400nm bei Raumtemperatur. Vorzugsweise ist das Absorptionsmaximum von Wasser bei 1470 nm +/-20 nm, besonders bevorzugt bei 1470 nm +/- 10 nm, besonders bevorzugt bei 1470 nm +/-5 nm. Vorzugsweise ist das Absorptionsmaximum von Kollagen bei 1500 nm +/- 20 nm, besonders bevorzugt bei 1500 nm +/- 10 nm, besonders bevorzugt bei 1500 nm +/- 5 nm. Vorzugsweise ist das Absorptionsmaximum von Fett bei 1210 und bei 1400 nm +/- 20 nm, besonders bevorzugt bei 1210 und bei 1400 nm +/- 10 nm, besonders bevorzugt bei 1210 und bei 1400 nm +/- 5 nm.

Vorzugsweise weist das Gewebeerkennungsverfahren ferner den Schritt auf:
- Speichern wenigstens einer Referenz in Form eines Absorptionsmaximums bei einer bestimmten Temperatur in der Recheneinheit, vorzugsweise einem Speichermedium in der Recheneinheit, vorzugsweise für Wasser und/oder Fett und/oder Kollagen.

Vorzugsweise kann mittels der Recheneinheit anhand des charakteristischen Absorptionsspektrums von Wasser als Referenz ermittelt werden, welche Temperatur in dem Gewebe herrscht. Auf der Recheneinheit bzw. dem Speichermedium ist gespeichert, dass Wasser bei einer bestimmten Temperatur ein bestimmtes Absorptionsmaximum (z.B. bei Raumtemperatur 1470 nm) hat. Durch vergleichen der Verschiebung der Absorptionsmaxima von einem vorgespeicherten Wert und/oder durch vergleichen mit einer Vielzahl vorgegebener korrespondierender Werte in einer gespeicherten Tabelle kann ermittelt werden, bei welcher Wellenlänge des Absorptionsmaximums welche Temperatur in dem Wasser des Gewebes herrscht. Das charakteristische Absorptionsspektrum von Wasser kann am einfachsten ermittelt werden, da die Gewebebestandteile im Körper bekannt sind und Wasser mit ca. 67% am stärksten im Gewebe vorhanden ist. Aufgrund des gemessenen Absorptionsspektrums kann die Verschiebung des spektralen Absorptionsmaximums von Wasser berechnet/ermittelt werden. Anhand dieser Verschiebung des Absorptionsmaximums, die ca. 0,5nm/K beträgt, kann die Temperatur bestimmt werden. Das vorstehende ist analog anwendbar auf Fett und/oder Kollagen und/oder anderen Bestandteilen des Gewebes.

Die vorstehenden Schritte zur Messung des Absorptionsspektrums können neben Wasser auch für Fett, Kollagen oder anderen Gewebebestandteilen analog angewendet werden. Somit können aus einem Absorptionsspektrum, das von einem Detektor erfasst und von einer Recheneinheit ermittelt wird, die einzelnen Absorptionsspektren von Wasser, Fett und Kollagen in Gewebe ermittelt werden.

Vorzugsweise weist das Gewebeerkennungsverfahren ferner den Schritt auf:
- Aufbringen der Beleuchtung und des Detektors auf das Gewebe. Vorteilhafterweise sind der Detektor und die Beleuchtung somit in direktem Kontakt mit dem Gewebe.

Vorzugsweise weist das Gewebeerkennungsverfahren ferner den Schritt auf:
- Steuern und/oder Regeln und/oder Abschalten einer Vorrichtung, vorzugsweise eines medizinischen Instruments, mittels der Recheneinheit auf Basis der berechneten Temperatur und/oder Gewebeimpedanz.

Vorzugsweise findet das Steuern und/oder Regeln und/oder Abschalten bei Erreichen einer vorbestimmten Temperatur statt, vorzugsweise bei einer Temperatur die größer 85° Celsius und kleiner 110°Celsius ist, besonders bevorzugt bei einer Temperatur die größer 95° Celsius und kleiner 100°Celsius ist. Die Koagulation von Gewebe erreicht das beste Ergebnis bei einer Temperatur, vorzugsweise konstanten Temperatur, bei einer Temperatur die größer 85° Celsius und kleiner 110°Celsius ist, besonders bevorzugt bei einer Temperatur die größer 95° Celsius und kleiner 100°Celsius ist.

Vorzugsweise finden alle Schritte online/ in Echtzeit statt. Das bedeutet, dass das Steuern und/oder das Regeln und/oder das Abschalten des medizinischen Instruments online, vorzugsweise in Echtzeit stattfindet. **In** anderen Worten ausgedrückt wird das Absorptionsspektrum des Gewebes online, vorzugsweise in Echtzeit gemessen, wodurch die Temperatur in dem Gewebe online, also in Echtzeit berechnet werden kann. Die Temperatur fließt dann vorzugsweise online, vorzugsweise in Echtzeit, in die Steuerung/ Regelung wenigstens einer Elektrode/ Sonotrode/ Laserquelle des medizinischen Instruments, vorzugsweise der Cut&Seal-Vorrichtung, mit ein.

Vorzugsweise wird das Gewebeerkennungsverfahren zur Temperaturmessung während eines Sealingvorgangs durchgeführt, besonders bevorzugt im Gewebe im medizinischen Instrument.

Vorzugsweise sind die Detektoren vorgesehen und angepasst Remission, vorzugsweise die Remissionsspektren, im NIR-Bereich von 1000nm bis 1700nm, besonders bevorzugt im Bereich von 1400nm bis 1600nm zu erfassen.

Vorzugsweise sind die wenigstens eine Beleuchtung und der wenigstens eine Detektor beabstandet, in einem medizinischen Instrument.

Vorzugsweise findet ein Verfahren zur Messung einer Gewebetemperatur Anwendung in einem medizinischen Instrument.

Vorzugsweise weist eine Temperaturmessvorrichtung ein Speichermedium auf, auf dem wenigstens einer der folgenden Schritte gespeichert ist (bei einer Mehrzahl vorzugsweise in dieser Reihenfolge):
- Speichern wenigstens einer Referenz in Form eines Absorptionsmaximums bei einer bestimmten Temperatur in der Recheneinheit, vorzugsweise einem Speichermedium in der Recheneinheit, vorzugsweise für Wasser und/oder Fett und/oder Kollagen.
- Aufbringen der Beleuchtung und des Detektors auf das Gewebe.Vorteilhafterweise sind der Detektor und die Beleuchtung somit in direktem Kontakt mit dem Gewebe.

- Aussenden von Licht, mit einem Beleuchtungsspektrum, vorzugsweise im VIS-/NIR-Bereich, in ein Gewebe mittels wenigstens einer Beleuchtung,
- Empfangen der Remission des Lichtes, mit einem Remissionsspektrum, aus dem Gewebe durch wenigstens einen Detektor, vorzugsweise einem Sensor,
- Umwandeln des Remissionsspektrums mittels des Detektors in ein Detektorsignal, vorzugsweise ein elektrisches Signal/Datensignal,
- Senden des Detektorsignals an eine Recheneinheit, vorzugsweise eine CPU,
- Berechnen des Remissionsspektrums aus dem Detektorsignal mittels der Recheneinheit,
- Berechnen eines Absorptionsspektrums des Gewebes durch Vergleich des Beleuchtungsspektrum mit dem Remissionsspektrum mittels der Recheneinheit,
- Berechnen wenigstens eines Absorptionsmaximums aus dem Absorptionsspektrum mittels der Recheneinheit,
- Berechnen einer Temperatur in dem Gewebe durch Vergleich des Absorptionsmaximums mit wenigstens einer, vorzugsweise in der Recheneinheit abgespeicherten, Referenz mittels der Recheneinheit, und
- Steuern und/oder Regeln und/oder Abschalten einer Vorrichtung, vorzugsweise eines medizinischen Instruments, mittels der Recheneinheit auf Basis der berechneten Temperatur und/oder Gewebeimpedanz.

In anderen Worten ausgedrückt werden in der Temperaturmessung während eines Sealingvorgangs online Remissionsspektren im NIR-Bereich von 1000nm bis 1700nm von einem Detektor erfasst. Die aus den aufgenommenen Spektren ableitbare Verschiebung der Lage der Absorptionsmaxima kann genutzt werden, um auf die Temperatur des im Instrument gefassten Gewebes, mit für die Anwendung ausreichender Genauigkeit, zu schließen, Mit zunehmender Temperatur verschiebt sich die Lage des Absorptionspeaks hin zu kürzeren Wellenlängen. Die Verschiebung ist dabei etwa 0,5nm/K. Kühlt das Gewebe weiter ab, verschiebt sich der Absorptionspeak wieder in Richtung längerer Wellenlängen. Da der Hauptabsorber im zu sealenden Gewebe im Wellenlängenbereich um ca. 1470 nm Wasser ist, spiegelt die so bestimmte Temperatur die Temperatur im Wasseranteil des Gewebes wieder. Der besondere Vorteil dieses Temperaturmessverfahrens ist, dass damit die tatsächliche Temperatur im Gewebe gemessen werden kann, da die NIR-Strahlung aufgrund der Streuung die gesamte Dicke der Gewebeschicht passieren kann. Im Gegensatz dazu wird bei der Messung der Temperatur während des Sealings mit einem Thermoelement lediglich die Temperatur der Kontaktfläche gemessen. Die Temperatur und die Wärmekapazität der Elektroden stellt für die Bestimmung der Gewebetemperatur bei dieser Methode eine Störgröße dar. Diese führt zu Latenzzeiten und Verfälschungen der wahren Gewebetemperatur. Diese Methode spiegelt somit nicht die Gewebetemperatur wider, sondern repräsentiert die Temperatur der Umgebung, mit der das Thermoelement in Kontakt ist. Mit der optischen Temperaturbestimmung ist es möglich wichtige Parameter für die Steuerung des Sealingprozesses zu gewinnen. Weiter kann die ermittelte Temperatur als Abschalt- /Regelungs-/Steuer-Kriterium/ Prozessparameter verwendet werden bzw. zur Prozessregelung/-Prozesssteuerung.

Es hat sich gezeigt, dass Licht vorzugsweise bestimmter Wellenlänge (z.B. Weißlicht im VIS-NIR-Bereich) vom Körpergewebe remittiert wird, wobei sich das Spektrum des vom Körpergewebe remittierten Lichts temperaturabhängig ändert. Es ist also möglich, eine Beleuchtung/Beleuchtungsausgang zur Bestrahlung von Körpergewebe sowie einen Detektor/Detektoreingang zur Erfassung von vom Körpergewebe remittiertem Licht unmittelbar an die Elektrode(n) heranzuführen und so die Gewebetemperatur in unmittelbarer Nähe zu (zwischen) der (den) Elektrode(n) über den Umweg des erfassten remittierten Lichts und dessen spektraler Verteilung zu bestimmen.

In der bevorzugten Ausführungsform hat ein medizinisches Instrument (der HF-Bauart) demzufolge
- wenigstens eine Instrumentenbranche, die wenigstens eine bestrombare Elektrode zum Versiegeln und/oder Schneiden von Gewebe bildet oder in oder an der wenigstens einen bestrombaren Elektrode zum Versiegeln und/oder Schneiden von Gewebe angeordnet ist, wobei die Bestromung der Elektrode durch eine Recheneinheit steuerbar und/oder regelbar ist, und
- wenigstens eine Temperaturmessvorrichtung, mit wenigstens einer Beleuchtung und wenigstens einem Licht-Detektor, die jeweils in oder an der zumindest einen Instrumentenbranche oder in Gegenüberlage in/an zwei Instrumentenbranchen (wechselweise) ausgebildet oder angeordnet ist/sind und die in elektrischer Verbindung mit der Recheneinheit stehen. In anderen Worten, sind auf einer Instrumentenbranche abwechselnd jeweils eine Beleuchtung und ein Detektor angeordnet, wobei auf der gegenüberliegenden Instrumentenbranche der Beleuchtung eine Beleuchtung und dem Detektor ein Detektor gegenüberliegt.

Eine Mehrzahl von Detektoren sind gemäß einem Aspekt abwechselnd an zwei sich gegenüberliegenden Instrumentenbranchen ausgebildet oder angeordnet und stehen in elektrischer Verbindung mit einer Recheneinheit. Dies bedeutet in anderen Worten, dass auf einer Instrumentenbranche abwechselnd jeweils eine Beleuchtung und ein Detektor angeordnet sind, wobei auf der gegenüberliegenden Instrumentenbranche der Beleuchtung eine Beleuchtung und dem Detektor ein Detektor gegenüberliegt, um eine Messverfälschung am jeweiligen Detektor durch auf der gegenüberliegenden Seite eingetragenes Licht zu reduzieren oder zu vermeiden.

Das medizinische Instrument ist ein chirurgisches Instrument, ein monopolares Instrument, ein bipolares Instrument, ein elektrochirurgisches Instrument, eine chirurgische Klammer, eine chirurgische Klemme, eine chirurgische Zange, eine chirurgische Schere, ein Skalpell und/oder dergleichen. Besonders bevorzugt ist das medizinische Instrument ein Seal&Cut-Instrument, das vorgesehen und angepasst ist, Gewebe mittels HF-Technologie zu schneiden und gleichzeitig zu versiegeln. Monopolare Instrumente haben den Vorteil, dass dadurch, dass sie einschalig ausgeformt sind (nur eine einzige Instrumentenbranche), eine kompakte Bauform ermöglicht wird und somit geringere Kosten bei deren Herstellung. Bipolare Instrumente (zwei gegenüberliegende Instrumentenbranchen) haben den Vorteil, dass eine aufgelöste Analyse besser umsetzbar ist und dass sie variabler in der Umsetzung der Duplizierung sind.

Vorzugsweise ist die mindestens eine Instrumentenbranche als der Teil Ende eines medizinischen Instruments zu verstehen, dessen distaler Teil ein Instrumentenbranchenkörper bzw. ein Gewebseingriffsabschnitt (Branchenkörper) ist, der mit dem Gewebe in Kontakt bringbar ist, und dessen proximaler Teil als Betätigungs- oder Griffabschnitt ausgebildet sind. Weiter bevorzugt ist die mindestens eine Instrumentenbranche eine Maulteilbranche. Der Instrumentenbranchenkörper der zumindest einen Instrumentenbranche kann als Elektrode zum Versiegeln von Gewebe ausgebildet sein, vorzugsweise ist der Instrumentenbranchenkörper dabei einstückig/ aus einem einzigen Teil aus einem leitfähigen Metall oder Graphit. Alternativ kann die Elektrode in und/oder an und/oder auf der Instrumentenbranche ausgebildet/ angeordnet/ eingebettet sein, vorzugsweise ist in diesem Fall die Instrumentenbranchenkörper dabei aus einem Isolator bzw. elektrisch isolierenden Material.

Vorzugsweise weist das medizinische Instrument zwei sich gegenüberliegende Instrumentenbranchen auf, die vorzugsweise gegeneinander bewegbaren/ schwenkbar sind, an deren Enden einander zugewandten Seiten/ Backen/ Bereiche/ Instrumentenbranchenenden/Instrumentenbranchenkörper angeordnet/ausgebildet sind, die mit dem Gewebe in Kontakt bringbar sind. Die Instrumentenbranchenenden/Instrumentenbranchenkörper können selbst als Elektroden zum Versiegeln von Gewebe ausgebildet sein, vorzugsweise sind die Instrumentenbranchenenden/Instrumentenbranchenkörper dabei aus einem leitfähigen Metall oder Graphit und gegeneinander isoliert. Die Elektroden können aber auch in und/oder an und/oder auf den Instrumentenbranchenenden/Instrumentenbranchenkörper ausgebildet/ angeordnet/ eingebettet sein, vorzugsweise sind die Instrumentenbranchenenden/Instrumentenbranchenkörper dabei aus einem Isolator bzw. elektrisch isolierenden Material oder sind aus Metall und gegen die Elektroden isoliert.

Vorzugsweise ist mindestens eine Elektrode durch die Recheneinheit steuerbar und/oder regelbar. Genauer gesagt ist die Stromstärke, die Spannung, die Phase und/oder die Frequenz des elektrischen Stroms, der/die an der Elektrode anliegt, steuerbar oder regelbar.

Vorzugsweise ist die Temperaturmessvorrichtung eine optische Temperaturmessvorrichtung/ ein Thermometer mit einem optischen Sender in Form einer Beleuchtung und einem optischen Empfänger in Form eines Licht-Detektors.

Vorzugsweise ist unter Beleuchtung wenigstens eine Lichtquelle/ Anregungslichtquelle und alternativ zusätzlich andere optische Bauteile zu verstehen, wie zum Beispiel ein Lichttunnel, der Lichtwellenleiter/ Spiegel/ Linsen/ reflektierende Innenwände/ streuende Medien und dergleichen aufweist. Weiter bevorzugt ist unter Lichtquelle eine Weißlichtquelle/ eine LED (im VIS- und/oder IR- und/oder UV-Bereich), eine Deuteriumlampe (UV-Bereich) und/oder eine Halogenlampe (VIS-Bereich) zu verstehen. In anderen Worten ausgedrückt kann das Licht an/in/auf der Instrumentenbranche an dem Einstrahlort/ an der wenigstens einen Eintrittsöffnung direkt mittels einer Lichtquelle erzeugt werden oder indem das Licht von einer Lichtquelle mittels Lichtwellenleiter/ Spiegel/ Linsen/ Lichttunnel/ streuende Medien und dergleichen an einen Einstrahlort/ eine Lichteinlassöffnung/ eine Lichteintrittsöffnung der Kontaktfläche der Instrumentenbranche, die vorgesehen und angepasst ist mit dem Gewebe in Kontakt zu kommen, geführt wird. Weiter bevorzugt erfolgt die Einstrahlung des Lichts der Beleuchtung in einem bestimmten Winkel relativ zu der Gewebekontaktfläche der entsprechenden Instrumentenbranche bzw. Elektrode, das heißt die Beleuchtung weist in/ an/ auf der Instrumentenbranche eine gewinkelte/ schräggestellte Austrittsöffnung und/oder Lichtstrahlung auf. In wieder anderen Worten ist die Lichtquelle selbst schräg/angewinkelt auf/an/in der Instrumentenbranche angeordnet oder weist eine schräge/ gewinkelte Oberfläche bezüglich der Gewebekontaktfläche bzw. Lichtaustrittsfläche auf. Alternativ kann ein optisches Element wie zum Beispiel ein Spiegel und/oder ein Lichtwellenleiter schräg auf/an/in der Kontaktoberfläche (der Fläche die vorgesehen und angepasst ist mit Gewebe in Kontakt zu kommen) der Instrumentenbranche angeordnet sein und das Licht von der Lichtquelle zu dem Einstrahlort bzw. der Kontaktfläche führen.

Eine Weißlichtquelle, also eine Lichtquelle, die elektromagnetische Strahlung über den ganzen VIS-Bereich aussendet, hat den Vorteil, dass mehr Information aus dem zu beleuchtenden Gewebe gewonnen werden kann, wodurch eine Gewebeerkennung und/oder eine multivariate Datenanalyse möglich sind. Des Weiteren besteht die Möglichkeit, eine Vielzahl verschiedener Messungen durchzuführen. Beispielsweise kann auf der Instrumentenbranche wenigstens eine Beleuchtung mit einer Weißlichtquelle und wenigstens ein Detektor angeordnet sein der vorgesehen und angepasst ist, spektrale Bereiche zu messen, vorzugsweise mit verschiedenen Sensoren (Si-, InGaAs-Sensoren usw.).

Eine Lichtquelle mit geringer spektraler Bandbreite hat den Vorteil, dass die Umsetzung einfach erfolgt, dass eine solche Lichtquelle kostengünstig ist, dass eine hohe zeitliche Abtastung mit einer solchen Lichtquelle erreicht werden kann und dass Abstände von mehr als 2 mm voneinander und/oder zu einem Detektor möglich sind, da eine höhere Intensität auf einen bestimmten Spektralbereich möglich ist.

Vorzugsweise ist unter Detektor bzw. Licht-Detektor wenigstens ein Sensor/ eine Photodiode und/oder ein Photomultiplier (PMT) und ggf. andere optische Bauteile zu verstehen, wie zum Beispiel ein Lichttunnel, der Lichtwellenleiter/ Spiegel/ Linsen/ / reflektierende Innenwände/ streuende Medien und dergleichen aufweisen kann. In anderen Worten ausgedrückt kann das Licht von dem in/an/auf der Instrumentenbranche eingebauten Detektor/ Detektorteil an dem Remissionsort direkt mittels eines dort angeordneten Sensors des Detektors oder dergleichen an/in/auf der Instrumentenbranche gemessen werden oder über einen Lichttunnel, der Lichtwellenleiter/ Spiegel/ Linsen/ / reflektierende Innenwände/ streuende Medien und dergleichen aufweisen kann und Licht von der Kontaktfläche/einer Lichteintrittsöffnung der Instrumentenbranche zu einem entfernt von der Kontaktfläche der Instrumentenbranche oder gar entfernt von der Instrumentenbranche angeordneten Sensor oder dergleichen geführt werden. Weiter bevorzugt erfolgt die Einstrahlung des Lichts ausgehend von der Beleuchtung in einem bestimmten Winkel (0° < Winkel ≤ 90°) relativ zu der Gewebekontaktfläche der entsprechenden Instrumentenbranche bzw. Elektrode. Weiter bevorzugt weist der Detektor in/an/auf der Instrumentenbranche eine zur Kontaktfläche ebenfalls gewinkelte/ schräggestellte Eintrittsöffnung auf. In wieder anderen Worten ist der Detektor selbst schräg/angewinkelt auf/an/in der Instrumentenbranche angeordnet oder weist eine schräge/ gewinkelte Oberfläche bezüglich der Gewebekontaktfläche auf. Alternativ kann ein optisches Element wie zum Beispiel ein Spiegel und/oder ein Lichtwellenleiter schräg auf/an/in der Kontaktoberfläche (der Fläche, die vorgesehen und angepasst ist mit Gewebe in Kontakt zu kommen) der Instrumentenbranche angeordnet sein und Remissionslicht zu einem entfernten Sensor oder dergleichen führen. Das nach der Einstrahlung vom Körpergewebe remittierte Licht wird vorzugsweise in mindestens zwei Kanäle spektral aufgelöst (mittels Spektrometer, Prismen oder unterschiedlichen Filtern) und dann von den mindestens zwei Sensoren oder dergleichen erfasst, die in Abhängigkeit hiervon mindestens zwei Signale zu der Recheneinheit/CPU senden, welche die mindestens zwei Signal in einen Temperaturwert transformiert.

Die Elektrode zum Versiegeln von Gewebe ist vorzugsweise aus Metall, leitfähiger Keramik, metallisierter Keramik, Graphit oder metallisiertem Graphit. Die Elektrode ist weiter vorzugsweise mit einer Oberfläche ausgebildet, die vorgesehen und angepasst ist, elektromagnetische Strahlung zu reflektieren.

Die Recheneinheit weist vorzugsweise einen Prozessor und ein Speichermedium auf. Das Speichermedium ist vorgesehen und angepasst, Schritte zur Ausführung der Messung der Temperatur und/oder der Steuerung und/oder Regelung des Stroms der Elektrode zu speichern.

Die Recheneinheit steuert die Beleuchtung/Lichtquelle der Beleuchtung (Dauer, Intensität, Wellenlänge usw.) mittels eines ersten elektrischen Signals und der Detektor erfasst das (ausschließlich) vom Körpergewebe gestreute/ reflektierte Licht bzw. die Remission unmittelbar an dem zu messenden/ behandelnden Gewebe (zwischen den Instrumentenbranchen) und sendet die ermittelten Daten als ein zweites elektrisches Signal an die Recheneinheit. Die Recheneinheit berechnet nun mittels eines Algorithmus auf dem Speichermedium, die aus dem jeweiligen zweiten elektrischen Signal ableitbare Temperatur des Gewebes. Auf Basis der so berechneten Temperatur des Gewebes wird online/ in Echtzeit berechnet welche Stromstärke, welche Spannung und/ oder welche Frequenz der elektrische Strom haben soll, welcher an die wenigstens eine Elektrode angelegt wird.

Zusätzlich kann in einer Ausführungsform auch der Widerstand des Gewebes (Gewebeimpedanz) von der Recheneinheit ermittelt werden und in die Berechnung mit einfließen. **In** anderen Worten kann die Gewebeimpedanz des Gewebes an/ zwischen den Elektroden/ Sonotroden ermittelt werden, sodass die Stromstärke, Spannung und/oder Frequenz des an die Elektrode(n), bzw. des US-Wandlers angelegten elektrischen Stroms in Erwiderung auf die ermittelte Gewebeimpedanz und (in Kombination mit) dem zweiten Signal der (optischen) Temperaturmessvorrichtung von der Recheneinheit gesteuert oder geregelt werden kann.

Vorzugsweise steht die Recheneinheit mit der erfindungsgemäßen (optischen) Temperaturmessvorrichtung so in Verbindung, dass die Stromstärke, die Spannung und/ oder die Frequenz des elektrischen Stroms, der an der wenigstens einen Elektrode anliegt in Antwort auf die von der Recheneinheit/CPU berechnete Temperatur änderbar ist, vorzugsweise automatisch und/oder durch einen vorgegebenen Algorithmus.

Vorzugsweise entspricht das zweite elektrische Signal von dem Detektor einem Lichtspektrum, das die Wellenlänge und die Intensität des am Detektor erfassten Lichts darstellt. Aufgrund dieses Spektrum wird die Verschiebung des spektralen Absorptionsmaximums von Wasser berechnet/ermittelt. Anhand dieser Verschiebung des Absorptionsmaximums, die ca. 0,5nm/K beträgt, kann die Temperatur bestimmt werden. Da das Absorptionsspektrum von Wasser charakteristisch ist, kann die Verschiebung auch ohne Referenzmessung und/oder mit Referenzmessung ermittelt werden.

Vorzugsweise ist die Recheneinheit so konfiguriert, dass sie wenigstens einen der folgenden Schritte aufweist oder es ist auf einem Speichermedium in der Recheneinheit wenigstens einer der folgenden Schritte gespeichert (vorzugsweise in der folgenden Reihenfolge):
- Ansteuern der Beleuchtung durch die Recheneinheit mit einem ersten elektrischen Signal, vorzugsweise mit einem elektrischen Strom mit bestimmter Stromstärke und/oder einer bestimmten Spannung und/oder einer bestimmten Frequenz,
- Aussenden einer elektromagnetischen Strahlung der der Beleuchtung (vorzugsweise Weißlicht) in das Gewebe, in einem bestimmten Bereich in unmittelbarer Nähe zu einer Elektrode oder zwischen zwei sich gegenüberliegenden Elektroden,
- Messen (mittels des Detektors) der Remission/ der diffusen Reflexion der elektromagnetischen Strahlung ausgehend vom Körpergewebe,
- Senden der Messergebnisse von dem Detektor an die Recheneinheit mittels eines zweiten elektrischen Signale,
- Transformieren des zweiten elektrischen Signals in einen Gewebetemperaturwert,
- Vorzugsweise Ermitteln der Gewebeimpedanz, vorzugsweise zwischen zwei Elektroden, und
- Verarbeiten des Gewebetemperaturwerts und vorzugsweise der ermittelten Gewebeimpedanz mittels der Recheneinheit, vorzugsweise mittels eines vorprogrammierten Algorithmus auf dem Speichermedium, zur Bestimmung einer neuen Stromstärke, Spannung und/oder Frequenz für den an die Elektrode(n) angelegten elektrischen Strom zur Erreichung oder Annäherung einer Temperatur des Gewebes von über 95° Celsius und bevorzugt gleichzeitig von unter 100°Celsius.

In einer Ausführungsform kann der Lichttunnel, der mit der Lichtquelle in Verbindung steht, an wenigstens einem Ende von wenigstens einer Lichtquelle gespeist werden und das wenigstens eine andere Ende in der Instrumentenbranche enden. In anderen Worten ausgedrückt kann Licht von wenigstens einer Lichtquelle über einen Lichtwellenleiter oder dergleichen an wenigstens einen Ausgang geleitet werden, der sich an/auf/in der Instrumentenbranche befindet. Alternativ kann sich wenigstens eine Lichtquelle, z.B. die LED, direkt auf/an/in der Instrumentenbranche befinden/angeordnet sein.

In einer Ausführungsform kann der Lichttunnel, der mit dem Detektor in Verbindung steht, an wenigstens einem Ende wenigstens einen Sensor aufweisen und an dem wenigstens einen anderen Ende in der Instrumentenbranche enden. In anderen Worten ausgedrückt kann Licht/Remission von wenigstens einem Eingang, der sich auf/in der Instrumentenbranche befindet, über einen reflektierenden Lichtkanal/ einen Lichtwellenleiter oder dergleichen an wenigstens einen Sensor/ eine Photodiode/ einen Photomultiplier oder dergleichen geleitet werden. Alternativ kann sich wenigstens ein Sensor/ Photodiode/ Photomultiplier auf/an/in der Instrumentenbranche befinden/angeordnet sein.

Vorzugsweise können sich die Beleuchtung und der Detektor ein Ende eines Lichttunnels teilen. In anderen Worten ausgedrückt können sich der Strahlengang der Lichtquelle und der Strahlengang des Sensors/ Photodiode/ Photomultipliers einen Lichttunnel teilen, so dass beides über eine einzige optische Öffnung, die gleichzeitig den Eingang und den Ausgang des Lichts auf/an/in der Instrumentenbranche bildet, mit dem Körpergewebe in optischem Kontakt ist.

Vorzugsweise sind eine Mehrzahl von Detektoren und eine Mehrzahl von Beleuchtungen auf wenigstens einer Instrumentenbranche angeordnet. Dabei können die Detektoren bzw. Beleuchtungen jeweils auf einer Instrumentenbranche in einem vorgegebenen Muster angeordnet sein. Das Muster ist vorzugsweise linienförmig. Alternativ kann/können wenigstens ein Detektor und/oder eine Beleuchtung auf einer ersten Instrumentenbranche angeordnet sein und wenigstens ein Detektor und/oder eine Beleuchtung auf einer zweiten Instrumentenbranche angeordnet sein, vorzugsweise auf einander zugewandten Seiten von gegenüberliegenden Instrumentenbranchen. In anderen Worten ausgedrückt kann in dieser Ausführungsform für bipolare Instrumente das Licht von einer Beleuchtungseinrichtung in das Gewebe eingebracht werden und auf einer gegenüberliegenden Seite kann ein Detektor das vom Gewebe remittierte Licht messen.

Vorzugsweise beträgt die Distanz zwischen der wenigstens einen Beleuchtung und dem wenigsten einen Detektor zwischen 0 und 5 mm, besonders bevorzugt zwischen 0 und 1 mm, da dort die Intensität der Remission sehr hoch ist.

Vorzugsweise weist die wenigstens eine Instrumentenbranche pro Beleuchtung mehrere Detektoren auf, besonders bevorzugt sind die Detektoren in gleichen und/oder unterschiedlichen Abständen zu der Beleuchtung angeordnet. In anderen Worten ausgedrückt kann der Abstand von einer Beleuchtung zu einem zweiten Detektor größer sein als der Abstand zu einem ersten Detektor.

Vorzugsweise weist die Beleuchtung eine diskrete Lichtquelle auf, vorzugsweise mit einer definierten Bandbreite, besonders bevorzugt mit einer Bandbreite kleiner 100 nm.

Vorzugsweise ist die (optische) Temperaturmessvorrichtung auf einer Ebene der Instrumentenbranche angeordnet, die tiefer liegt als die Kontaktfläche der Elektrode. In anderen Worten ausgedrückt bildet eine Kontaktfläche der Elektroden und/oder der Instrumentenbranchen, die mit Gewebe in Kontakt kommt, eine Ebene aus. Diese Ebene liegt in Kontaktrichtung höher (näher am Gewebe) als die Ebene, auf der die wenigstens eine Beleuchtung und oder der wenigstens eine Detektor angeordnet ist.

Vorzugsweise ermöglicht die (optische) Temperaturmessvorrichtung eine Echtzeit/ online-Bestimmung der Temperatur während eines Sealingvorgangs/ Sealings. Die Online-Bestimmung ist von besonderer Bedeutung für die Güte des Sealings. Die Messung repräsentiert dabei die Temperatur im Gewebe/ die Gewebetemperatur und weist keine Latenzzeit auf oder eine Verfälschung der gemessenen Temperatur durch die Wärmekapazität der Messvorrichtung, beispielsweise durch die Wärmekapazität von Elektroden aus Metall. Der Vorteil einer optischen Temperaturmessung, die sensitiv auf das Wasser im gefassten/ im in Kontakt kommenden Gewebe ist, liegt darin, dass diese Temperaturmessvorrichtung keine nennenswerte Wärmekapazität hat.

Vorzugsweise kann die Remissionsmessung in der Instrumentenbranche bzw. in dem Maulteil eines Seal&Cut Instruments unabhängig von der Position, an der das Gewebe mit der Instrumentenbranche in Kontakt kommt, durchgeführt werden. In anderen Worten ausgedrückt ist die Temperaturmessvorrichtung auf der Oberfläche der Instrumentenbranche in dem Bereich, der dazu vorgesehen und angepasst ist mit dem Gewebe in Kontakt zu kommen, verteilt angeordnet, vorzugsweise gleichmäßig verteilt. Wie vorstehend dargelegt kann die wenigstens eine Instrumentenbranche eine Vielzahl von Anrege- und Detektionspfaden/ Beleuchtungs- oder Detektionspfaden aufweisen, vorzugsweise entlang und/oder in einer Elektrode.

Wie vorstehend ausgeführt wurde soll zusätzlich oder alternativ zu der Messung der Impedanz, eine Messung der Temperatur erfolgen. Die Temperatur wird direkt im zu fusionierenden Gewebe vorzugsweise zwischen zwei sich gegenüberliegenden Instrumentenbranchen gemessen und zwar vorzugsweise im (zeitlichen) Verlauf der Bestromung/Erwärmung des Gewebes. Hierdurch kann die Veränderung des Gewebezustands direkt/online detektiert und somit auch darauf reagiert werden. Durch die Erweiterung des Algorithmus durch einen weiteren Steuer-/ Regelparameter ist es möglich den Energieeintrag in das Gewebe besser zu bewerten und somit die Fusion des Gewebes besser zu steuern/regeln. Zudem können mit der erfindungsgemäßen Temperaturmessvorrichtung auch andere Eigenschaften des Gewebes gemessen werden, zum Beispiel der Wasseranteil/ der Wassergehalt im Gewebe.

Vorzugsweise weist die Elektrode auf der Fläche, die vorgesehen und angepasst ist, mit dem Gewebe in Kontakt zu kommen, wenigstens eine erste Elektrodenoberfläche auf. Die Elektrode befindet sich vorzugsweise auf einem Instrumentenbranchenkörper (im Maulteil) einer Instrumentenbranche oder wird durch die Instrumentenbranche gebildet. In der Elektrode und/oder der Instrumentenbranche sind vorzugsweise wenigstens eine Lichtquelle/ wenigstens ein Lichtleiter/ wenigstens ein optisches Bauteil (dichroitischer Spiegel/ Strahlungsteiler/ Spiegel) und/oder wenigstens ein Lichtdetektor (oder ein Teil davon) mit wenigstens einem Sensor und ggf. einem Lichtleiter eingebracht. Als Sensor kann auch eine Photodiode oder ein Photomultiplier verstanden werden. Die Elektrode weist vorzugsweise wenigstens eine Lichtaustrittsöffnung auf, aus/durch die das Licht der Lichtquelle aus der Elektrodenoberfläche und/oder in das Gewebe strahlt. Die Elektrode weist vorzugsweise wenigstens eine Lichteintrittsöffnung auf, durch die das Licht (ausschließlich) aus dem Gewebe (Remission) in/durch die Elektrodenoberfläche in den Sensor strahlt/ remittiert/ reflektiert wird. Die Elektrode weist vorzugsweise wenigstens einen Kanal auf, der vorgesehen und angepasst ist, Daten mittels wenigstens eines Kabels/elektrischen Leitung an wenigstens eine Recheneinheit zu leiten, oder Licht, mittels wenigstens eines streuenden Mediums/ wenigstens eines Lichtwellenleiter/ wenigstens einer spiegelnden Oberfläche an einen entfernt gelegenen Sensor zu leiten, der wiederum Daten, mittels wenigstens eines Kabels/elektrischen Leitung an wenigstens eine Recheneinheit leitet. Wenn die Erfindung mehr als eine Elektrodenoberfläche bzw. mehr als eine Instrumentenbranche aufweist, sind die Elektrodenoberflächen/Instrumentenbranchen voneinander beabstandet, vorzugsweise parallel. Der Raum zwischen den Elektrodenoberflächen/Instrumentenbranchen ist vorzugsweise vorgesehen und angepasst, eine Schneidvorrichtung, wie ein Messer, Skalpell, HF-Skalpell oder dergleichen, einschiebbar aufzunehmen, die vorgesehen und angepasst ist, Gewebe zu trennen/schneiden. Auf den wenigstens zwei Seiten des Schnitts des Gewebes sind somit die Elektroden-/Branchenoberflächen ausgeformt, um das Gewebe mittels HF-Technik zu Koagulieren.

Vor dem Sensor ist vorzugsweise ein schmalbandiger Filter angeordnet. Ein Lichttunnel kann in der Elektrode und/oder der Instrumentenbranche gebildet werden. In anderen Worten ausgedrückt kann der Lichttunnel Licht durch die Instrumentenbranche und/oder die wenigstens eine Elektrode führen. Alle Ausführungsformen können untereinander kombinierbar sein.

Die Erfindung wird nachstehend anhand bevorzugter Ausführungsbeispiele unter Bezugnahme auf die begleitenden Zeichnungen näher erläutert.

### Figurenbeschreibung

Fig. 1 zeigt einen Bereich einer Instrumentenbranche gemäß einer ersten Ausführungsform.
Fig. 2 zeigt eine erste Beleuchtungs- und Detektionsanordnung einer Instrumentenbranche.
Fig. 3 zeigt eine zweite Beleuchtungs- und Detektionsanordnung einer Instrumentenbranche.
Fig. 4 zeigt eine dritte Beleuchtungs- und Detektionsanordnung einer Instrumentenbranche.
Fig. 5 zeigt einen Bereich einer Instrumentenbranche gemäß einer zweiten Ausführungsform.
Fig. 6 zeigt die Lichtführung in dem Bereich der Instrumentenbranche gemäß der zweiten Ausführungsform.
Fig. 7 zeigt einen Bereich einer Instrumentenbranche gemäß einer dritten Ausführungsform.
Fig. 8 zeigt die Lichtführung in dem Bereich der Instrumentenbranche gemäß der dritten Ausführungsform.
Fig. 9 zeigt einen Bereich einer Instrumentenbranche gemäß einer vierten Ausführungsform.
Fig. 10 zeigt die Lichtführung in dem Bereich der Instrumentenbranche gemäß der vierten Ausführungsform.
Fig. 11 zeigt einen Bereich einer Instrumentenbranche gemäß einer fünften Ausführungsform.
Fig. 12 zeigt die Lichtführung in dem Bereich der Instrumentenbranche gemäß der fünften Ausführungsform.
Fig. 13 zeigt einen Bereich einer Instrumentenbranche gemäß einer sechsten Ausführungsform.
Fig. 14 zeigt die Lichtführung in dem Bereich der Instrumentenbranche gemäß der sechsten Ausführungsform.
Fig. 15 zeigt einen Bereich einer Instrumentenbranche gemäß einer siebten Ausführungsform.
Fig. 16 zeigt die Lichtführung in dem Bereich der Instrumentenbranche gemäß der sechsten Ausführungsform.
Fig. 17 zeigt einen Bereich einer Instrumentenbranche gemäß einer achten Ausführungsform.
Fig. 18 zeigt die Lichtführung in dem Bereich der Instrumentenbranche gemäß der achten Ausführungsform.
Fig. 19 zeigt einen Bereich einer Instrumentenbranche gemäß einer neunten Ausführungsform.
Fig. 20 zeigt die Lichtführung in dem Bereich der Instrumentenbranche gemäß der neunten Ausführungsform.
Fig. 21 zeigt einen Bereich einer Instrumentenbranche gemäß einer zehnten Ausführungsform.
Fig. 22 zeigt die Lichtführung in dem Bereich der Instrumentenbranche gemäß der zehnten Ausführungsform.
Fig. 23 zeigt eine bipolare Instrumentenbranche gemäß vorstehenden Ausführungsformen.
Fig. 24 zeigt gegenüberliegende Detektoren und Beleuchtungen auf einem bipolaren HF-Instrument.
Fig. 25 zeigt eine schematische Darstellung einer medizinischen Vorrichtung gemäß der Erfindung.
Fig. 26 zeigt ein Beispiel eines medizinischen Hochfrequenz-Chirurgie-Instruments gemäß der Erfindung.

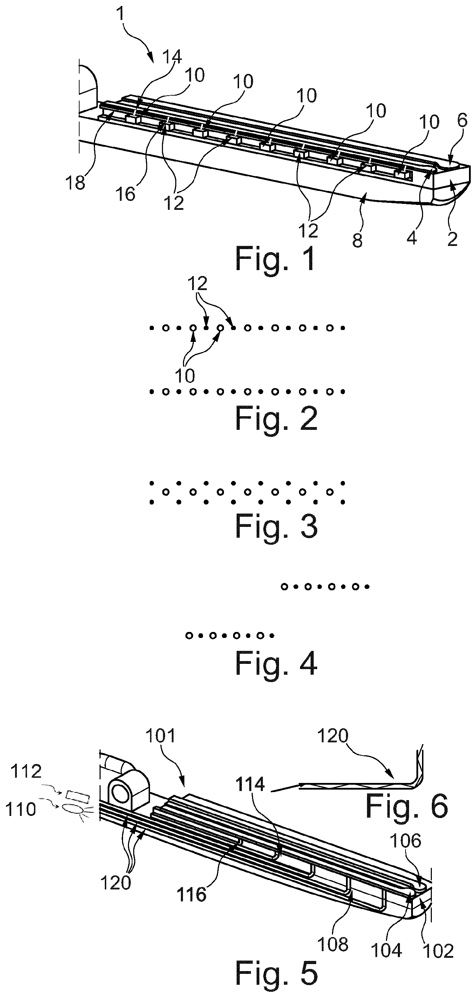

Fig. 1 zeigt einen Bereich einer Instrumentenbranche 1 gemäß einer ersten Ausführungsform. Die Instrumentenbranche 1 weist mindestens eine Elektrode 2 auf, welche in die Instrumentenbranche 1 in isolierter Weise eingebettet ist. Die Elektrode 2 weist auf der Branchenseite, die vorgesehen und angepasst ist, mit einem Körpergewebe in Kontakt zu kommen, eine erste Elektrodenoberfläche 4 und eine zweite Elektrodenoberfläche 6 auf. Die Elektrode(n) 2 befindet sich insbesondere in/auf einem Instrumentenbranchenkörper 8 der Instrumentenbranche 1, welcher eine Hälfte eines betätigbaren Instrumentenmaulteils darstellt. In die Elektrode 2 bzw. in die Instrumentenbranche 1/den Instrumentenbranchenkörper 8 sind abwechselnd Lichtquellen (LED's) 10 und Lichtdetektoren bzw. Sensoren 12 eingebracht. Die Elektrode 2 bzw. die Instrumentenbranche 1/der Instrumentenbranchenkörper 8 weist Lichtaustrittsöffnungen 14 auf, durch die das Licht der Lichtquelle 10 aus der Elektrodenoberfläche 4 und/oder 6 bzw. der Branchenkontaktfläche in das Gewebe strahlt. Die Elektrode 2 bzw. die Instrumentenbranche 1/der Instrumentenbranchenkörper 8 weist ferner Lichteintrittsöffnungen 16 auf, durch die das Licht aus dem Gewebe in/ durch die Elektrodenoberfläche 4 und/oder 6 bzw. durch die Branchenkontaktfläche in den Sensor 12 remittiert wird. Die Elektrode 2 bzw. die Instrumentenbranche 1/der Instrumentenbranchenkörper 8 weist wenigstens einen (Längs-)Kanal 18 auf, der vorgesehen und angepasst, ist Daten/Signale von den Sensoren 12 mittels eines Kabels (nicht näher dargestellt) an eine Recheneinheit (nicht näher dargestellt) zu leiten.

Fig. 2 zeigt eine erste Variante einer Beleuchtungs- und Detektionsanordnung der Instrumentenbranche 1. Jede der Ausführungsformen dieser Anmeldung kann die erste Beleuchtungs- und Detektionsanordnung aufweisen. Die obere Reihe der Beleuchtungs- und Detektionsanordnung der Figur 2 ist an/in der zweiten Elektroden-/Branchenoberfläche 6 der Fig. 1 angeordnet/eingebettet. Die untere Reihe der Beleuchtungs- und Detektionsanordnung der Figur 2 ist an/in der ersten Elektroden-/Branchenoberfläche 4 der Fig. 1 angeordnet/eingebettet. In den Reihen ist jeweils abwechselnd ein Detektor/ Sensor 12 und eine Beleuchtung/ Lichtquelle 10 angeordnet. Die dunklen Punkte stellen dabei einen Detektor/Sensor 12 und die hellen Punkte eine Beleuchtung/Lichtquelle 10 das. Vor dem Detektor/Sensor 12 ist vorzugsweise ein schmalbandiger (Licht-)Filter (nicht dargestellt) angeordnet. Weiter vorzugsweise sind die optoelektronischen Bauteile (Sensor 12 und Beleuchtung 10) auf einer Platine unterhalb der Elektrode/unterhalb der Gewebekontaktfläche der Branche angebracht.

Fig. 3 zeigt eine zweite Variante einer Beleuchtungs- und Detektionsanordnung einer Instrumentenbranche. Jede der Ausführungsformen dieser Anmeldung kann die zweite Variante einer Beleuchtungs- und Detektionsanordnung aufweisen. Die dunklen Punkte stellen dabei einen Sensor 12 und die hellen Punkte eine Lichtquelle 10 dar. Die zweite Variante einer Beleuchtungs- und Detektionsanordnung ist so ausgestaltet, dass um eine Lichtquelle 10 jeweils vier Sensoren 12 in einem gleichen Abstand zur Lichtquelle 10 angeordnet sind, wobei sich die eine Lichtquelle10 jeweils zwei Sensoren 12 mit einer anderen, unmittelbar benachbarten Lichtquelle teilt. Die/jede Lichtquelle 10 befindet sich in anderen Worten im Mittelpunkt eines imaginären Rechtecks, an dessen Eckpunkten die Sensoren 12 positioniert sind.

Fig. 4 zeigt eine dritte Variante einer Beleuchtungs- und Detektionsanordnung einer Instrumentenbranche. Jede der Ausführungsformen dieser Anmeldung kann die dritte Variante einer Beleuchtungs- und Detektionsanordnung aufweisen. Die dunklen Punkte stellen dabei einen Sensor 12 und die hellen Punkte eine Lichtquelle 10 dar. Die dritte Variante einer Beleuchtungs- und Detektionsanordnung ist gleich der ersten Variante einer Beleuchtungs- und Detektionsanordnung mit dem Unterschied, dass die Reihe der Beleuchtungs- und Detektionsanordnung der zweiten Elektroden-/Branchenoberfläche dort anfängt, wo die die Reihe der Beleuchtungs- und Detektionsanordnung der ersten Elektroden-/Branchenoberfläche endet.

Fig. 5 zeigt einen Bereich einer Instrumentenbranche 101 gemäß einer zweiten Ausführungsform. Die Instrumentenbranche 101 weist eine Elektrode 102 auf. Die Elektrode 102 weist auf der (Branchen-)Fläche, die vorgesehen und angepasst ist, mit dem Gewebe in Kontakt zu kommen, eine erste Elektrodenoberfläche 104 und eine zweite Elektrodenoberfläche 106 auf. Insofern entspricht die Branche der zweiten Ausführungsform der Branche der ersten Ausführungsform. Die Elektrode 102 befindet sich insbesondere auf einem distalen Instrumentenbranchenkörper 108 der Instrumentenbranche 101, der einen Teil eines Instrumenten-Maulteils darstellt. In die Instrumentenbranche 101, zum Beispiel in einem Betätigungsabschnitt oder in einem Griffabschnitt der Instrumentenbranche 101, sind Lichtquellen 110 und Sensoren 112 (nicht näher dargestellt) entfernt zur Gewebekontaktfläche des Instrumentenbranchenkörpers 108 eingebracht. Die Elektrode 102/ Instrumentenbranchenkörper 108 weist Lichtaustrittsöffnungen 114 auf, durch die das Licht der Lichtquelle geleitet wird und aus denen Licht aus der Elektrodenoberfläche 104 und/oder 106 bzw. Gewebekontaktfläche des Instrumentenbranchenkörpers 108 in das Gewebe strahlt/ eintritt. Die Elektrode 102/Instrumentenbranchenkörper 108 weist Lichteintrittsöffnungen 116 auf, durch die das Licht aus dem Gewebe in/ durch die Elektrodenoberfläche 104 und/oder 106 bzw. Gewebekontaktfläche des Instrumentenbranchenkörpers 108 in einen Lichttunnel 120 strahlt/ eintritt, der in dem Sensor endet. Auch das Licht von der Lichtquelle zu der Lichtaustrittsöffnung 114 wird durch einen, vorzugsweise anderen, Lichttunnel 120 geleitet. Die Lichttunnel 120 sind mit Luft oder einem anderen Gas gefüllt oder weisen ein Vakuum auf. Die Lichttunnel 120 führen durch den Instrumentenbranchenkörper 108 und/oder durch die Elektrode 102. Die, vorzugsweise zylindrischen Lichttunnel 120 weisen eine innere Tunneloberfläche (in der hohlzylindrischen Form) auf, die wiederum reflektierende Eigenschaften für elektromagnetische Wellen (Lichtwellen) aufweist. Die Tunneloberfläche an der Tunnel-Innenseite ist somit vorgesehen und angepasst, eine Totalreflexion zu ermöglichen.

Fig. 6 zeigt die Lichtführung in dem Bereich der Instrumentenbranche/des Instrumentenbranchenkörpers gemäß der zweiten Ausführungsform in dem Lichttunnel 120. Das eintretende Licht von der Lichtquelle kommend wird an der Innenoberfläche des Lichttunnels 120 totalreflektiert und kann somit durch den Lichttunnel 120 geleitet werden. Durch die Totalreflexion an der Innenseite des Lichttunnels 120 kann das Licht auch durch abgebogene Bereiche/ wenigstens einen Bogen oder dergleichen geführt werden. In diesem Fall ist der Lichttunnel 120 längs des Branchenkörpers 108 geführt um dann in einem im Wesentlichen 90°-Bogen zur Gewebekontaktfläche des Branchenkörpers 108 (oder einem anderen Winkel bezüglich der Gewebekontaktfläche) zu gelangen, wo sich der Lichttunnel 120 öffnet.

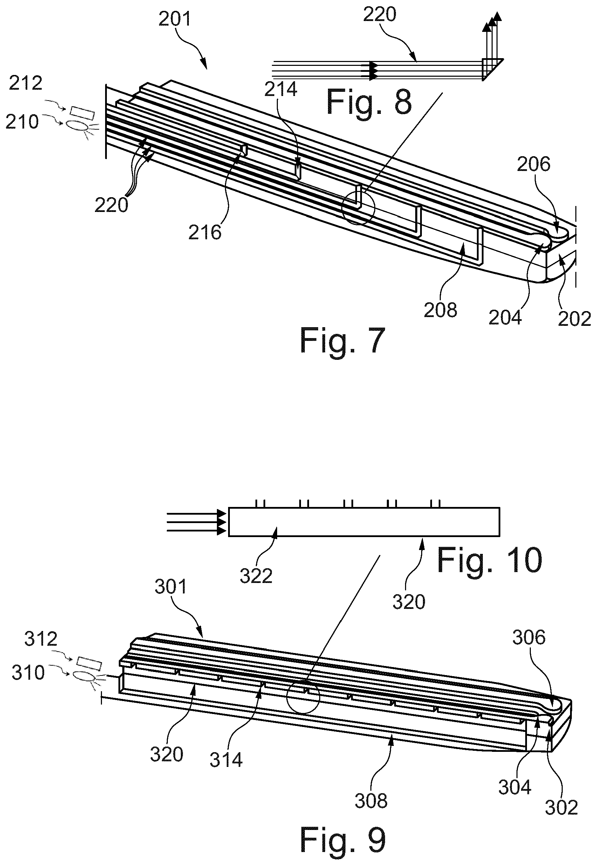

Fig. 7 zeigt einen Bereich einer Instrumentenbranche 201 gemäß einer dritten Ausführungsform. Die Instrumentenbranche 201 weist einen Instrumentenbranchenkörper 208 auf, der ein Teil eines Instrumentenmaulteils bildet, der eine Elektrode ist oder in welchem, wie in der Fig. 7 gezeigt, eine Elektrode 202 isolierend eingebettet ist. Die Elektrode 202 weist auf der Branchenfläche, die vorgesehen und angepasst ist, mit dem Gewebe in Kontakt zu kommen, eine erste Elektrodenoberfläche 204 und eine zweite Elektrodenoberfläche 206 auf. Die Elektrode 202 befindet sich demzufolge auf/in dem Instrumentenbranchenkörper 208 der Instrumentenbranche 201. In die Instrumentenbranche 201, zum Beispiel in einem Betätigungsabschnitt oder in einem Griffabschnitt der Instrumentenbranche 201, sind Lichtquellen 210 und Sensoren 212 entfernt zur Gewebekontaktfläche eingebracht (nicht näher dargestellt). Die Elektrode 202 bzw. der Instrumentenbranchenkörper 208 weist Lichtaustrittsöffnungen 214 auf, durch die das Licht der nicht dargestellten Lichtquelle geleitet wird und aus denen Licht aus der Elektrodenoberfläche 204 und/oder 206 bzw. der Gewebekontaktfläche in das Gewebe strahlt/ eintritt. Die Elektrode 202 bzw. der Instrumentenbranchenkörper 208 weist Lichteintrittsöffnungen 216 auf, durch die das Licht aus dem Gewebe in/ durch die Elektrodenoberfläche 204 und/oder 206 bzw. die Gewebekontaktfläche des Instrumentenbranchenkörpers 208 in einen Lichttunnel 220 strahlt/ eintritt, der in einem Sensor endet. Auch das Licht von der Lichtquelle zu der Lichteintrittsöffnung 216 wird durch einen, vorzugsweise anderen, Lichttunnel 220 geleitet. Die Lichttunnel 220 sind mit Luft oder einem anderen Gas gefüllt oder weißen ein Vakuum auf. Die Lichttunnel 220 führen durch den Instrumentenbranchenkörper 208 und/oder durch die Elektrode 202. Das Licht der Lichtquelle wird senkrecht zur Öffnung des, vorzugsweise zylindrischen, Lichttunnels 220/ zur Längsrichtung des zylindrischen Lichttunnels 220 eingeleitet/ eingestrahlt. Das Licht wird somit gerade/ geradlinig in dem Lichttunnel 220 geführt. Zur Lenkung des Lichts wird wenigstens ein Spiegel und/oder ein Prisma im Lichttunnel 220 verwendet, um das Licht in einem gewünschten Winkel abzulenken/ zu führen. Der Tunnel 220 kann jedwede geometrische Form annehmen, z.B. zylindrisch, quaderförmig, usw..

Fig. 8 zeigt die Lichtführung in dem Bereich der Instrumentenbranche gemäß der dritten Ausführungsform in dem Lichttunnel 220. Das eintretende Licht von der Lichtquelle kommend wird geradlinig/ gerichtet/ parallel gerichtet in den Lichttunnel 220 eingespeist. Durch die Führung mittels wenigstens einem Spiegel in dem Lichttunnel 220 kann das Licht auch über gewinkelte Beiche/ Winkel oder dergleichen geführt werden.

Fig. 9 zeigt einen Bereich einer Instrumentenbranche 301 gemäß einer vierten Ausführungsform. Die Instrumentenbranche 301 weist eine Elektrode 302 auf, die in einem Instrumentenbranchenkörper 308 aufgenommen ist, der eine Gewebekontaktfläche ausbildet. Die Elektrode 302 weist auf der Fläche, die vorgesehen und angepasst ist, mit dem Gewebe in Kontakt zu kommen, eine erste Elektrodenoberfläche 304 und eine zweite Elektrodenoberfläche 306 auf. Die Elektrode 302 befindet sich somit in/auf dem Instrumentenbranchenkörper 308 der Instrumentenbranche 301. **In** der Instrumentenbranche 301, zum Beispiel in einem Betätigungsabschnitt oder in einem Griffabschnitt der Instrumentenbranche 301 sind entfernt zur Gewebekontaktfläche des Instrumentenbranchenkörpers 308 Lichtquellen und Sensoren eingebracht (nicht näher dargestellt). Die Elektrode 302 bzw. der Instrumentenbranchenkörper 308 weist Lichtaustrittsöffnungen 314 auf, durch die das Licht der nicht dargestellten Lichtquelle geleitet wird und aus denen Licht aus der Elektrodenoberfläche 304 und/oder 306 bzw. bzw. dem Instrumentenbranchenkörper 308 in das Gewebe strahlt/ eintritt. Die Elektrode 302 bzw. der Instrumentenbranchenkörper 308 weist Lichteintrittsöffnungen (nicht gezeigt) auf, durch die das Licht aus dem Gewebe in/ durch die Elektrodenoberfläche 304 und/oder 306 bzw. durch die Kontaktfläche des Instrumentenbranchenkörpers 308 in einen Lichttunnel 320 strahlt/ eintritt, der in einem Sensor endet. Auch das Licht von der Lichtquelle zu der Lichtaustrittsöffnung 314 wird durch einen, vorzugsweise anderen, Lichttunnel (nicht dargestellt) geleitet. Die Lichttunnel 320 sind mit einem streuenden Bulkmaterial 322 gefüllt. Die Lichttunnel 320 führen durch den Instrumentenbranchenkörper 308 und/oder durch die Elektrode 302. Bei dieser Ausführungsform sind wenigstens zwei Lichttunnel 320 parallel in der Elektrode 302 und/ oder dem Instrumentenbranchenkörper 308 in einer Reihe/ Linie angeordnet, so dass jeweils eine Reihe mit Lichteintrittsöffnungen 314 und Lichtaustrittsöffnungen (nicht gezeigt) in jeweils eine Elektrodenoberfläche 304 und 306 eingebracht ist. In einer nicht gezeigten Ausführungsform kann das Bulk-Material der vierten Ausführungsform selbst eine Lichtquelle darstellen, d.h. das Bulkmaterial kann leuchten.

Fig. 10 zeigt die Lichtführung in dem Bereich der Instrumentenbranche gemäß der vierten Ausführungsform in einem Lichttunnel 320. Das eintretende Licht von der Lichtquelle kommend wird in den Lichttunnel 320 eingespeist, genauer gesagt in das streuende und/oder leuchtende Bulkmaterial 322 in dem Lichttunnel 320. Durch die Streuung des Lichts in dem Bulkmaterial 322 wird das Licht in das Gewebe gestrahlt und das remittierte Licht wird von einem anderen Lichttunnel (nicht gezeigt) mit dem gleichen Aufbau zu dem Sensor geleitet/gestreut.

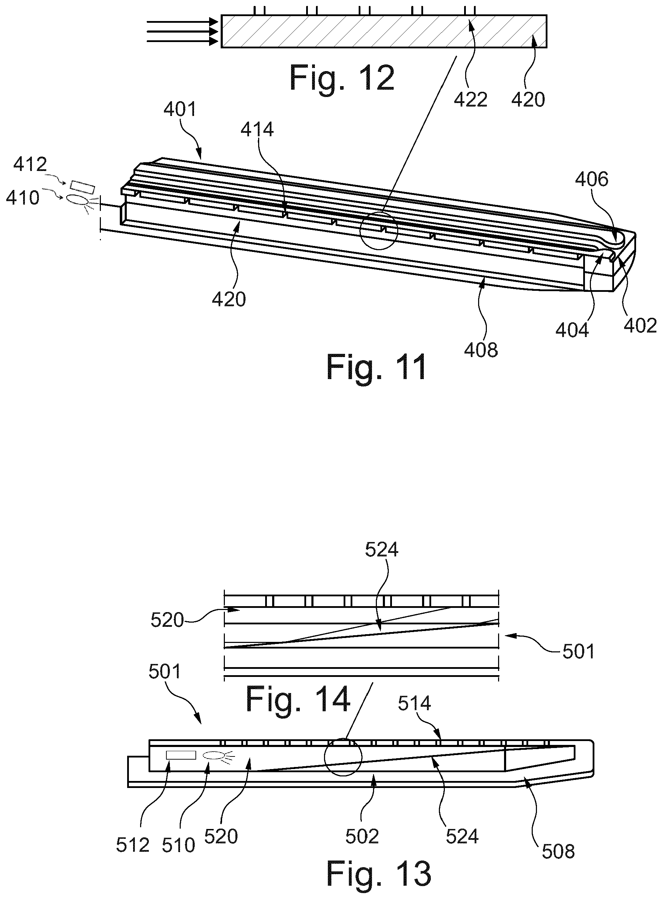

Fig. 11 zeigt einen Bereich einer Instrumentenbranche 401 gemäß einer fünften Ausführungsform. Die Instrumentenbranche 401 weist eine Elektrode 402 auf, die vorliegend in einem Instrumentenbranchenkörper 408 isolierend eingebettet ist. Die Elektrode 402 weist auf der Fläche des Instrumentenbranchenkörpers 408, die vorgesehen und angepasst ist, mit dem Gewebe in Kontakt zu kommen, eine erste Elektrodenoberfläche 404 und eine zweite Elektrodenoberfläche 406 auf. Die Elektrode 402 befindet sich somit in/auf dem Instrumentenbranchenkörper 408 der Instrumentenbranche 401. In die Instrumentenbranche 401, zum Beispiel in einem Betätigungsabschnitt oder in einem Griffabschnitt der Instrumentenbranche 401, sind Lichtquellen 410 und Sensoren 412 entfernt zur Gewebekontaktfläche des Instrumentenbranchenkörpers 408 eingebracht (nicht näher dargestellt). Die Elektrode 402 bzw. der Instrumentenbranchenkörper 408 weist Lichtaustrittsöffnungen 414 auf, durch die das Licht einer nicht dargestellten Lichtquelle geleitet wird und aus denen Licht aus der Elektrodenoberfläche 404 und/oder 406 bzw. aus der Gewebekontaktfläche in das Gewebe strahlt/ eintritt. Die Elektrode 402 bzw. der Instrumentenbranchenkörper 408 weist Lichteintrittsöffnungen (nicht gezeigt) auf, durch die das Licht aus dem Gewebe in/ durch die Elektrodenoberfläche 404 und/oder 406 bzw. durch die Gewebekontaktfläche in einen Lichttunnel 420 strahlt/ austritt, der in einem Sensor endet. Auch das Licht von der Lichtquelle zu der Lichtaustrittsöffnung 414 wird durch einen, vorzugsweise anderen, Lichttunnel (nicht dargestellt) geleitet. Die Lichttunnel 420 sind mit einem strukturierten Bulkmaterial 422 gefüllt. Die Lichttunnel 420 führen durch den Instrumentenbranchenkörper 408 und/oder durch die Elektrode 402. Bei dieser Ausführungsform sind wenigstens zwei Lichttunnel 420 parallel in der Elektrode 402 und/ oder dem Instrumentenbranchenkörper 408 in einer Reihe/ Linie angeordnet, so dass jeweils eine Reihe mit Lichteintrittsöffnungen 414 und Lichtaustrittsöffnungen (nicht gezeigt) in jeweils eine Elektrodenoberfläche 404 und 406 eingebracht ist. In einer nicht gezeigten Ausführungsform kann das Bulk-Material der fünften Ausführungsform selbst eine Lichtquelle darstellen, d.h. das Bulkmaterial kann leuchten.

Fig. 12 zeigt die Lichtführung in dem Bereich der Instrumentenbranche gemäß der fünften Ausführungsform in einem Lichttunnel 420. Das eintretende Licht von der Lichtquelle kommend wird in den Lichttunnel 420 eingespeist, genauer gesagt in das strukturierte Bulkmaterial 422 in dem Lichttunnel 420. Durch die Struktur des Einsatzes in dem Bulkmaterial 422 wird das Licht in das Gewebe gestrahlt und das remittierte Licht wird von einem anderen Lichttunnel (nicht gezeigt) mit dem gleichen Aufbau zu einem Sensor geleitet/gestreut.

Fig. 13 zeigt einen Bereich einer Instrumentenbranche 501 gemäß einer sechsten Ausführungsform. Die Instrumentenbranche 501 weist eine Elektrode 502 auf, wobei in dieser Ausführungsform der Instrumentenbranchenkörper 501 und die Elektrode 502 hinsichtlich ihres Aufbaus und Anordnung den vorhergehenden Ausführungsbeispielen entsprechen. In dem Instrumentenbranchenkörper 508 sind Lichtquellen 510 und Sensoren 512 eingebracht (nicht näher dargestellt). Die Elektrode 502/ der Instrumentenbranchenkörper weist Lichtaustrittsöffnungen 514 auf, durch die das Licht einer Lichtquelle geleitet wird und aus denen Licht in das Gewebe strahlt/ eintritt. Die Elektrode 502/ der Instrumentenbranchenkörper weist Lichteintrittsöffnungen (nicht gezeigt) auf, durch die das Licht aus dem Gewebe in einen Lichttunnel 520 strahlt/ eintritt, der in einem Sensor endet. Das Licht von der Lichtquelle zu der Lichteintrittsöffnung 514 wird durch wenigstens einen Lichttunnel 520 geleitet. Bei dieser Ausführungsform ist ein einziger Lichttunnel 520 in der Elektrode 502 und somit in dem Instrumentenbranchenkörper 501 ausgebildet. Es ist jeweils eine Reihe mit Lichtaustrittsöffnungen 514 und Lichteintrittsöffnungen (nicht gezeigt) in die Elektrode 502 bzw. in den Instrumentenbranchenkörper eingebracht. In dem Lichttunnel 520 ist wenigstens eine verspiegelte/ reflektierende schiefe/ angewinkelte Ebene 524 ausgeformt. Die Ebene 524 kann durch Polieren der Elektrode bzw. des Instrumentenbranchenköpers hergestellt werden oder indem ein Spiegel in den Lichttunnel 520 eingebracht wird. Der Lichttunnel 520 führt durch den Instrumentenbranchenkörper. Es ist wenigstens eine Reihe mit Lichtaustrittsöffnungen 514 und Lichteintrittsöffnungen (nicht gezeigt) in einer Oberfläche der Elektrode 502 / des Instrumentenbranchenkörpers eingebracht. Alternativ oder zusätzlich kann ein einziger Lichttunnel 520 dieser Art sowohl für die Anregung als auch für die Aufnahme von reflektiertem Licht dienen - mit den entsprechenden Filtern. Das bedeutet, dass nach der Lichtquelle ein Filter angebracht ist, der dem Remissionswellenlängenbereich entspricht, das restliche Licht wird aber in das Gewebe geleitet und von derselben und/oder einer benachbarten Öffnung aufgenommen und über dieselbe reflektierende Ebene 524 zurück an den Sensor geführt.

Fig. 14 zeigt die Lichtführung in dem Bereich der Instrumentenbranche 501 gemäß der sechsten Ausführungsform in dem Lichttunnel 520. Das eintretende Licht von der Lichtquelle kommend wird in den Lichttunnel 520 eingespeist und an der angewinkelten, spiegelnden Ebene 524 in einem vorbestimmten Winkel (vorzugsweise mit einem Winkel zwischen 0° und 90°) abgelenkt. Durch den/ die Spiegel/ spiegelnde Oberfläche/ spiegelnde Ebene 524 wird das Licht in das Gewebe gestrahlt und das remittierte Licht wird von einem anderen Lichttunnel (nicht gezeigt) mit dem gleichen Aufbau zu einem Sensor geleitet/ geführt.

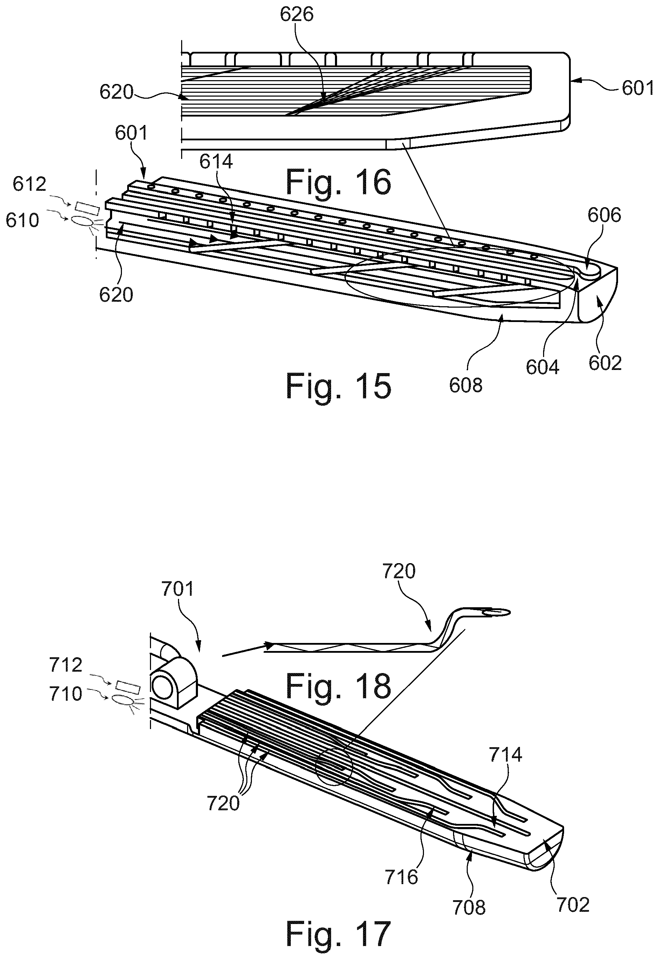

Fig. 15 zeigt einen Bereich einer Instrumentenbranche 601 gemäß einer siebten Ausführungsform. Die Instrumentenbranche 601 weist eine von einem Instrumentenbranchenkörper 608 aufgenommene Elektrode 602 auf. Die Elektrode 602 weist auf der Fläche des Instrumentenbranchenkörpers 608, die vorgesehen und angepasst ist, mit dem Gewebe in Kontakt zu kommen, eine erste Elektrodenoberfläche 604 und eine zweite Elektrodenoberfläche 606 auf. In dem Instrumentenbranchenkörper 608 sind Lichtquellen 610 und Sensoren 612 eingebracht (nicht näher dargestellt). Der Instrumentenbranchenkörper 608 weist Lichtaustrittsöffnungen 614 auf, durch die das Licht einer Lichtquelle 610 geleitet wird und aus der Gewebekontaktfläche in das Gewebe strahlt/ eintritt. Der Instrumentenbranchenkörper 608 weist ferner Lichteintrittsöffnungen 616 (nicht näher gezeigt) auf, durch die das Licht aus dem Gewebe in/ durch die Gewebekontaktfläche des Instrumentenbranchenkörpers 608 in einen Lichttunnel 620 strahlt/ eintritt, der in einem Sensor endet. Auch das Licht von der Lichtquelle zu der Lichtaustrittsöffnung 614 wird durch einen zweiten Lichttunnel (nicht dargestellt) geleitet. In den Lichttunnel 620 ist wenigstens eine teil-lichtdurchlässige Ebene 626 eingebracht, die einen Teil einer elektromagnetischen Strahlung transmittiert, also für einen Teil des Lichts durchlässig ist und ein Teil des Lichts reflektieren. Vorzugsweise ist die teil-lichtdurchlässige Ebene ein teil-lichtdurchlässiger Spiegel und weiter bevorzugt sind in dem Lichttunnel mehrere teil-lichtdurchlässige Ebenen 626 hintereinander angeordnet.

Fig. 16 zeigt die Lichtführung in dem Bereich der Instrumentenbranche 601 gemäß der siebten Ausführungsform in einem Lichttunnel 620. Das eintretende Licht von der Lichtquelle kommend wird in den Lichttunnel 620 eingespeist. Das eintretende Licht von der Lichtquelle kommend wird geradlinig/ gerichtet/ parallel gerichtet in den Lichttunnel 620 eingespeist. Durch die Führung mittels wenigstens eines teil-lichtdurchlässigen Spiegels 626 in dem Lichttunnel 620 wird das Licht über gewinkelte Bereiche/ Winkel oder dergleichen geführt/ reflektiert/ gespiegelt. Das Licht, das einen teil-lichtdurchlässigen Spiegel 626 durchdringt, trifft auf einen weiteren teil-lichtdurchlässigen Spiegel 626, der in dem gleichen Winkel wie der der vorherige Spiegel angeordnet ist und so weiter. Durch den teil-lichtdurchlässigen Spiegel/ spiegelnde Oberfläche/ spiegelnde Ebene 626 wird das Licht in das Gewebe gestrahlt und das remittierte Licht wird von einem anderen Lichttunnel (nicht gezeigt) mit dem gleichen Aufbau zu einem Sensor geleitet/ geführt.

Fig. 17 zeigt einen Bereich einer Instrumentenbranche 701 gemäß einer achten Ausführungsform. Die Instrumentenbranche 701 weist eine Elektrode 702 auf. Die Elektrode 702 befindet sich auf einem Instrumentenbranchenkörper 708 der Instrumentenbranche 701. In die Instrumentenbranche 701, zum Beispiel in einem Betätigungsabschnitt oder in einem Griffabschnitt der Instrumentenbranche 701, sind Lichtquellen 710 und Sensoren 712 entfernt zum Instrumentenbranchenkörper 708 eingebracht, vorzugsweise extern (nicht näher dargestellt). Der Instrumentenbranchenkörper 708 weist wenigstens einen Lichttunnel 720 auf, durch den das Licht der Lichtquelle geleitet wird und aus dem Licht in das Gewebe strahlt/ eintritt. Die Instrumentenbranchenkörper 708 weist wenigstens einen weiteren Lichttunnel 720 auf, durch den das Licht aus dem Gewebe zu einem Sensor geführt wird. Die Lichttunnel 720 werden in dieser Ausführungsform von Lichtwellenleitern wie zum Beispiel Glasfasern gebildet.

Fig. 18 zeigt die Lichtführung in dem Bereich der Instrumentenbranche gemäß der achten Ausführungsform in einem Lichttunnel 720. Das eintretende Licht von der Lichtquelle kommend wird an der Innenoberfläche des Lichttunnels 720 totalreflektiert und kann somit durch den Lichttunnel 720 geleitet werden. Durch die Totalreflexion an der Innenseite des Lichttunnels 720 kann das Licht auch durch abgebogene Bereiche/ wenigstens einen Bogen oder dergleichen geführt werden.

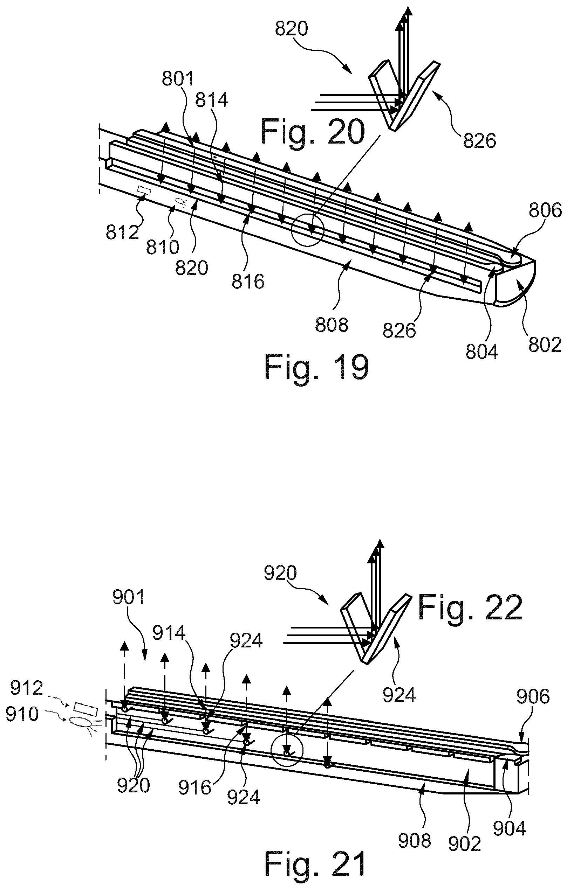

Fig. 19 zeigt einen Bereich einer Instrumentenbranche 801 gemäß einer neunten Ausführungsform. Die Instrumentenbranche 801 weist eine Elektrode 802 auf. Die Elektrode 802 weist auf der Gewebekontaktfläche ihres Instrumentenbranchenkörpers, die vorgesehen und angepasst ist, mit dem Gewebe in Kontakt zu kommen, eine erste Elektrodenoberfläche 804 und eine zweite Elektrodenoberfläche 806 auf. In dem Instrumentenbranchenkörper 808 sind Lichtquellen 810 und Sensoren 812 eingebracht (nicht näher dargestellt). Der Instrumentenbranchenkörper 808 weist zudem Lichtaustrittsöffnungen 814 auf, durch die das Licht einer Lichtquelle geleitet wird und in das Gewebe strahlt/ eintritt. Der Instrumentenbranchenkörper weist auch Lichteintrittsöffnungen 816 auf, durch die das Licht aus dem Gewebe in/ durch den Instrumentenbranchenkörper in einen Lichttunnel 820 strahlt/ eintritt, der in einem Sensor endet. Das Licht von der Lichtquelle zu der Lichtaustrittsöffnung 814 wird durch denselben Lichttunnel geleitet. In anderen Worten ausgedrückt, können Lichtaustrittsöffnungen 814 als Lichteintrittsöffnungen 816 fungieren und vice versa. In den Lichttunnel 820 sind wenigstens zwei teil-lichtdurchlässige Ebenen 626 eingebracht, die einen Teil einer elektromagnetischen Strahlung transmittieren, also für einen Teil des Lichts durchlässig sind, und einen Teil des Lichts reflektieren. Vorzugsweise ist die teildurchlässige Ebene ein teildurchlässiger Spiegel und weiter bevorzugt sind in dem Lichttunnel mehrere teildurchlässige Ebenen 626 hintereinander angeordnet. Durch diese Anordnung in dieser Ausführungsform ist jeweils ein teildurchlässiger Spiegel jeweils einer Lichtaustrittsöffnung 814 oder einer Lichteintrittsöffnung 816 zugeordnet.

Fig. 20 zeigt die Lichtführung in dem Bereich der Instrumentenbranche 801 gemäß der neunten Ausführungsform in einem Lichttunnel 820. Das eintretende Licht von der Lichtquelle kommend wird in den Lichttunnel 820 eingespeist. Das eintretende Licht von der Lichtquelle kommend wird geradlinig/ gerichtet/ parallel gerichtet in den Lichttunnel 820 eingespeist. Durch die Führung mittels wenigstens zweier teil-lichtdurchlässigen Spiegel 826 in dem Lichttunnel 820 wird das Licht über gewinkelte Bereiche/ Winkel oder dergleichen geführt/ reflektiert/ gespiegelt. Das Licht, das einen teil-lichtdurchlässigen Spiegel 826 durchdringt, trifft auf einen wenigstens einen weiteren teil-lichtdurchlässigen Spiegel 826, der in dem gleichen Winkel wie der vorherige Spiegel angeordnet ist und so weiter. Durch den teil-lichtdurchlässigen Spiegel/ spiegelnde Oberfläche/ spiegelnde Ebene 826 wird das Licht in das Gewebe gestrahlt und das remittierte Licht wird von demselben Lichttunnel 820 aber durch eine benachbarte Öffnung zu einem Sensor geleitet/ geführt. In wieder anderen Worten ist eine Öffnung zugleich Lichtaustrittsöffnung und Lichteintrittsöffnung für eine benachbarte Öffnung.

Fig. 21 zeigt einen Bereich einer Instrumentenbranche 901 gemäß einer zehnten Ausführungsform.

Die Instrumentenbranche 901 weist eine Elektrode 902 auf, wobei in dieser Ausführungsform der Instrumentenbranchenkörper sowie die Elektrode hinsichtlich ihres Aufbaus und Anordnung den vorhergehenden Ausführungsbeispielen entsprechen. In der Instrumentenbranche 901, zum Beispiel in einem Betätigungsabschnitt oder in einem Griffabschnitt der Instrumentenbranche 901, sind demzufolge Lichtquellen 910 und Sensoren 912 entfernt zu der Gewebekontaktfläche des Instrumentenbranchenkörpers eingebracht (nicht näher dargestellt). Der Instrumentenbranchenkörper 908 weist Lichtaustrittsöffnungen 914 auf, durch die das Licht einer Lichtquelle geleitet wird und aus denen Licht in das Gewebe strahlt/ eintritt. Der Instrumentenbranchenkörper weist ferner Lichteintrittsöffnungen 916 auf, durch die das Licht aus dem Gewebe in einen Lichttunnel 920 strahlt/ austritt, der in einem Sensor endet. Das Licht von der Lichtquelle zu der Lichtaustrittsöffnung 914 wird durch wenigstens einen Lichttunnel 920 geleitet. Das Licht von der Lichteintrittsöffnung 916 zu dem Sensor wird durch wenigstens einen weiteren Lichttunnel 920 (der gleichen Bauart) geleitet. Bei dieser Ausführungsform sind somit wenigstens zwei Lichttunnel 920 in dem Instrumentenbranchenkörper 908 ausgebildet. Die Lichtaustrittsöffnung/en 914 und Lichteintrittsöffnung/en 916 sind abwechselnd in den Instrumentenbranchenkörper eingebracht. In dem Lichttunnel 920 ist wenigstens eine verspiegelte/ reflektierende schiefe/ angewinkelte Ebene 924 ausgeformt.

Fig. 22 zeigt die Lichtführung in dem Bereich der Instrumentenbranche 901 gemäß der zehnten Ausführungsform in dem Lichttunnel 920. Das eintretende Licht von der Lichtquelle kommend wird in den Lichttunnel 920 eingespeist, und an der angewinkelten spiegelnden Ebene 924 in einem vorbestimmten Winkel (vorzugsweise mit einem Winkel zwischen 0 und 90°) abgelenkt. Durch den/ die Spiegel/ spiegelnde Oberfläche/ spiegelnde Ebene 924 wird das Licht in das Gewebe gestrahlt und das remittierte Licht wird von einem anderen Lichttunnel 920 mit dem gleichen Aufbau zu einem Sensor geleitet/ geführt.

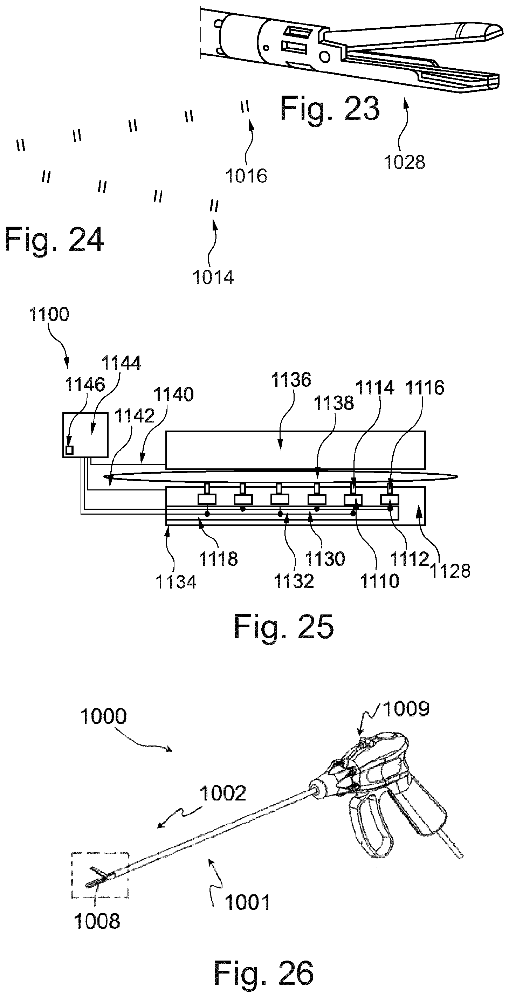

Fig. 23 zeigt eine bipolare Instrumentenbranche gemäß vorstehender Ausführungsformen. Die Ausführungsformen eins bis zehn sind vorgesehen und angepasst, in einem bipolaren medizinischen HF-Instrument verwendet zu werden, bei welchem zwei Instrumentenbranchenkörper vorzugsweise schwenkbar zueinander gelagert sind und zwischen sich einen Gewebeaufnahmespalt definieren.

Fig. 24 zeigt sich gegenüberliegende Detektoren und Beleuchtungen an einem bipolaren HF-Instrument. Dabei sind der die Lichtaustrittsöffnungen 1014 der Beleuchtungen und die Lichteintrittsöffnungen 1016 der Detektoren jeweils auf gegenüberliegenden Instrumentenbranchen/ Instrumentenbranchenkörpern angeordnet.

Fig. 25 zeigt eine schematische Darstellung einer medizinischen Vorrichtung 1100 gemäß der Erfindung. Eine Lichtquelle 1110 ist dafür vorgesehen und angepasst, Licht auszustrahlen. Ein Sensor 1112 ist dafür vorgesehen und angepasst, Licht zu detektieren. Die Lichtquelle strahlt das Licht durch eine Lichtaustrittsöffnung 1114. Der Sensor 1112 empfängt Licht über eine Lichteintrittsöffnung 1116. Die Lichtquellen 1110 und die Sensoren 1112 sind in Verbindung mit in einem Kanal 1118 befindlichen Datenleitungen 1130 und 1132. Der Kanal 1118 ist in einem Instrumentenbranchenkörper 1128 ausgebildet, die auch die Elektroden isolierend aufnimmt. Der einen Instrumentenbranchenkörper, in welcher die Elektrode 1134 aufgenommen ist, klemmt mit einem Instrumentenbranchenkörper, in welchem die gegenüberliegende Elektrode 1136 aufgenommen ist, das Gewebe 1138 ein. Die Elektrode 1134 und die Elektrode 1136 sind mit den Leitungen 1140 und 1142 in Verbindung. Die Datenleitungen 1130 und 1132, sowie die Leitungen 1140 und 1142 sind in Verbindung mit einer Recheneinheit 1144, die ein Speichermedium 1146 aufweist.

Fig. 26 zeigt ein Bespiel eines medizinischen Hochfrequenz-Chirurgie-Instrument 1000 gemäß der Erfindung, das eine erste Instrumentenbranche 1001 und eine zweite Instrumentenbranche 1002 aufweist. Am distalen Ende der ersten Instrumentenbranche 1001 ist ein Instrumentenbranchenkörper 1008 gebildet und am proximalen Ende der ersten Instrumentenbranche 1001 ist ein Betätigungs- oder Griffabschnitt 1009 gebildet.

### Bezugszeichenliste

1, 101, 201, 301, 401, 501, 601, 701, 801, 901, 1001, 1002 Instrumentenbranche
2, 102, 202, 302, 402, 502, 602, 702, 802, 902 Elektrode
4, 104, 204, 304, 404, 604, 804, 904 Erste Elektrodenoberfläche
6, 106, 206, 306, 406, 606, 806, 906 Zweite Elektrodenoberfläche
8, 108, 208, 308, 408, 508, 608, 708, 808, 908, 1008, 1128 Instrumentenbranchenkörper
10, 110, 210, 310, 410, 510, 610, 710, 810, 910, 1110 Lichtquelle
12, 112, 212, 312, 412, 512, 612, 712, 812, 912, 1112 Sensor
14, 114, 214, 314, 414, 514, 614, 714, 814, 914, 1014, 1114 Lichteintrittsöffnung
16, 116, 216, 616, 716, 816, 916, 1016, 1116 Lichtaustrittsöffnung
18, 1118 Kanal
120, 220, 320, 420, 520, 620, 720, 820, 920 Lichttunnel
322, 422 Bulk-Material
524, 924 Verspiegelte schiefe Ebene
626, 826 Teildurchlässige Eben
1028 Bipolare Instrumentenbranche
1100 Medizinische Vorrichtung
1130, 1132, 1140, 1142 Leitungen
1134 Erste Elektrode
1136 Zweite Elektrode
1138 Gewebe
1144 Recheneinheit
1146 Speichermedium
1000 Medizinisches Hochfrequenz-Chirurgie-Instrument
1009 Bestätigungs- oder Griffabschnitt

## Patentansprüche

1. Medizinisches Hochfrequenz-Chirurgie Instrument (1000, 1100) mit
- zumindest einer Instrumentenbranche (1, 101, 201, 301, 401, 501, 601, 701, 801, 901, 1001, 1002),
- zumindest einer Lichtquelle (10, 110, 210, 310, 410, 510, 610, 710, 810, 910, 1110), insbesondere einer LED, oder Lichtquellenbaugruppe, insbesondere eine LED und ein Filter, die ein erstes Licht mit einem bestimmten Beleuchtungs-Lichtspektrum erzeugt, welches in Richtung hin zu einem Gewebe aussendbar ist, und
- zumindest einem Sensor (12, 112, 212, 312, 412, 512, 612, 712, 812, 912, 1112), der dafür vorgesehen und angepasst ist, ein zweites Licht mit einem, vorzugsweise vom Beleuchtungs-Lichtspektrum unterschiedlichen, Remissionsspektrum zu erfassen, welches von dem Gewebe infolge der Lichtbeaufschlagung durch die Lichtquelle (10, 110, 210, 310, 410, 510, 610, 710, 810, 910, 1110) reflektiert wird, und
- das zweite Licht entsprechend dessen Remissionsspektrums in ein Detektorsignal umzuwandeln, wobei
eine Recheneinheit (1144) dazu vorgesehen und ausgebildet ist, um
- das Detektorsignal von dem zumindest einen Sensor (12, 112, 212, 312, 412, 512, 612, 712, 812, 912, 1112) zu empfangen,
- ein theoretisches Remissionsspektrum basierend auf einer Lösung zur Beschreibung der Lichtausstrahlung im Gewebe, vorzugsweise basierend auf der Strahlungstransporttheorie und ihrer Näherungen, mittels der Recheneinheit (1144), unter Annahme geschätzter Volumenanteile der einzelnen Gewebebestandteile, die im Gewebe vorhanden sind, zu berechnen,
- das theoretische Remissionsspektrum an das gemessene Remissionsspektrum beispielsweise durch eine nichtlineare Regression, ein Neuronales Netz oder eine Look-Up-Tabelle mittels der Recheneinheit (1144) anzupassen, und
- wenigstens einen Volumenanteil eines Gewebebestandteils aus dem Remissionsspektrum über einen Minimierungsalgorithmus zu berechnen, mit dem das theoretisch berechnete Remissionsspektrum an das gemessene Remissionsspektrum mittels der Recheneinheit (1144) durch Variation der Volumenanteile der einzelnen Gewebebestandteile, die im Gewebe vorhanden sind, angefittet (angepasst) wird,
- wenigstens ein Absorptionsmaximum aus einem Absorptionsspektrum des Gewebes zu berechnen, wobei das Absorptionsspektrum des Gewebes aus dem Fit des theoretisch berechneten Remissionsspektrums an das gemessene Remissionsspektrum ermittelt wird,
- eine Temperatur in dem Gewebe durch Vergleich des Absorptionsmaximums mit wenigstens einer Referenz zu berechnen,
- das medizinische Hochfrequenz-Chirurgie Instrument (1000, 1100) auf Basis des berechneten Volumenanteils eines Gewebebestandteils und der berechneten Temperatur, oder auf Basis des berechneten Volumenanteils eines Gewebebestandteils, der berechneten Temperatur und berechneter Gewebeimpedanz zu steuern und/oder zu regeln und/oder abzuschalten.

2. Medizinisches Hochfrequenz-Chirurgie Instrument (1000, 1100) nach Anspruch 1, das ferner dazu vorgesehen, angepasst und ausgebildet ist, um
- wenigstens eine Referenz in Form eines Absorptionsmaximums bei einer bestimmten Temperatur in der Recheneinheit (1144), vorzugsweise einem Speichermedium in der Recheneinheit (1144), vorzugsweise für Wasser und/oder Fett und/oder Kollagen, zu speichern.

3. Medizinisches Hochfrequenz-Chirurgie Instrument (1000, 1100) nach einem der vorstehenden Ansprüche, das ferner dazu vorgesehen, angepasst und ausgebildet ist, um
- die zumindest eine Lichtquelle (10, 110, 210, 310, 410, 510, 610, 710, 810, 910, 1110) und den zumindest einen Sensor (12, 112, 212, 312, 412, 512, 612, 712, 812, 912, 1112) auf das Gewebe aufzubringen.

4. Medizinisches Hochfrequenz-Chirurgie-Instrument (1000, 1100) nach Anspruch 1, **dadurch gekennzeichnet, dass** das medizinisches Hochfrequenz-Chirurgie-Instrument (1100) vorgesehen; angepasst und ausgebildet ist, um bei Erreichen einer vorbestimmten Temperatur zu steuern und/oder zu regeln und/oder abzuschalten, vorzugsweise bei einer Temperatur die größer 95° Celsius und Kleiner 100°Celsius stattfindet.

5. Medizinisches Hochfrequenz-Chirurgie-Instrument (1000, 1100) nach einem der vorstehenden Ansprüche, **dadurch gekennzeichnet, dass** das medizinische Hochfrequenz-Chirurgie-Instrument (1000, 1100) vorgesehen, angepasst und ausgebildet ist, um das medizinische Hochfrequenz-Chirurgie Instrument (1000, 1100) online zu steuern und/oder zu regeln und/oder abzuschalten, vorzugsweise in Echtzeit erfolgt.

6. Medizinisches Hochfrequenz-Chirurgie-Instrument (1000, 1100) nach einem der vorstehenden Ansprüche, **dadurch gekennzeichnet, dass** das medizinische Hochfrequenz-Chirurgie-Instrument (1000, 1100) dazu vorgesehen, angepasst und ausgebildet ist, um die Gewebeerkennung während eines Sealingvorgangs durchzuführen.

7. Medizinisches Hochfrequenz-Chirurgie-Instrument (1000, 1100) nach einem der vorstehenden Ansprüche, **dadurch gekennzeichnet, dass** der zumindest eine Sensor (12, 112, 212, 312, 412, 512, 612, 712, 812, 912, 1120) vorgesehen und angepasst ist, die Remission, vorzugsweise die Remissionsspektren, im NIR-Bereich von 1000nm bis 1700nm, besonders bevorzugt im Bereich von 1400nm bis 1600nm zu messen.

8. Medizinisches Hochfrequenz-Chirurgie-Instrument (1000, 1100) nach einem der vorstehenden Ansprüche, **dadurch gekennzeichnet, dass** die zumindest eine Lichtquelle (10, 110, 210, 310, 410, 510, 610, 710, 810, 910, 1100) und der zumindest eine Sensor (12, 112, 212, 312, 412, 512, 612, 712, 812, 912, 1120) beabstandet sind.

9. Medizinisches Hochfrequenz-Chirurgie-Instrument (1000, 1100) nach einem der vorstehenden Ansprüche, **dadurch gekennzeichnet, dass** die Instrumentenbranche (1, 101, 201, 301, 401, 501, 601, 701, 801, 901) einen Instrumentenbranchenkörper (8, 108, 208, 308, 408, 508, 608, 708, 808, 908, 1128), der eine Hälfte eines betätigbaren Instrumentenmaulteils des medizinischen Hochfrequenz-Chirurgie-Instruments (1000, 1100) ausbildet, und zumindest eine Elektrode (2, 102, 202, 302, 402, 502, 602, 702, 802, 902) aufweist, die vorgesehen und angepasst ist, mit dem Gewebe in Kontakt zu kommen und in oder auf dem Instrumentenbranchenkörper (8, 108, 208, 308, 408, 508, 608, 708, 808, 908, 1128) angeordnet ist, wobei
die zumindest eine Lichtquelle (10, 510, 810, 1100) und der zumindest ein Sensor (12, 512, 812, 1120) in oder auf dem Instrumentenbranchenkörper (8, 508, 808, 1128) angeordnet sind.

10. Medizinisches Hochfrequenz-Chirurgie-Instrument (1000, 1100) nach einem der vorstehenden Ansprüche 1 bis 8, **dadurch gekennzeichnet, dass** die Instrumentenbranche (1, 101, 201, 301, 401, 501, 601, 701, 801, 901, 1001, 1002) einen Instrumentenbranchenkörper (8, 108, 208, 308, 408, 508, 608, 708, 808, 908, 1128), der eine Hälfte eines betätigbaren Instrumentenmaulteils des medizinischen Hochfrequenz-Chirurgie-Instruments (1100) ausbildet, und zumindest eine Elektrode (2, 102, 202, 302, 402, 502, 602, 702, 802, 902) aufweist, die vorgesehen und angepasst ist, mit dem Gewebe in Kontakt zu kommen und in oder auf dem Instrumentenbranchenkörper (8, 108, 208, 308, 408, 508, 608, 708, 808, 908, 1128) angeordnet ist, wobei
zumindest ein Lichttunnel (120, 220, 320, 420, 620, 720, 820, 920) in oder auf der Instrumentenbranche (101, 201, 301, 401, 501, 601, 701, 801, 901, 1001, 1002) angeordnet ist, durch welchen das erste Licht von der zumindest einen Lichtquelle (110, 210, 310, 410, 510, 610, 710, 810, 910) und/oder das zweite Licht aus dem Gewebe geleitet wird.

11. Maschinenlesbares Speichermedium **gekennzeichnet durch** auf dem Speichermedium gespeicherte Schritte, die die Recheneinheit (1114) des medizinischen Hochfrequenz-Chirurgie Instruments (1000, 1100) nach einem der vorstehenden Ansprüche 1 bis 10 ausführt.

## Claims

1. A medical high frequency surgical instrument (1000, 1100) comprising
- at least one instrument branch (1, 101, 201, 301, 401, 501, 601, 701, 801, 901, 1001, 1002),
- at least one light source (10, 110, 210, 310, 410, 510, 610, 710, 810, 910, 1110), in particular an LED or a light source assembly, in particular an LED and a filter which generates a first light with a certain illumination light spectrum which can be emitted in the direction towards a tissue, and
- at least one sensor (12, 112, 212, 312, 412, 512, 612, 712, 812, 912, 1112), which is provided and adapted to detect a second light with a remission spectrum, preferably different from the illumination light spectrum, and which is reflected by the tissue as a result of light impingement by the light source (10, 110, 210, 310, 410, 510, 610, 710, 810, 910, 1110), and
- to convert the second light into a detector signal according to its remission spectrum, wherein
a calculating unit (1144) is provided and adapted to
- receive the detector signal from the at least one sensor (12, 112, 212, 312, 412, 512, 612, 712, 812, 912, 1112),
- to calculate a theoretical remission spectrum based on a solution for describing the light emission in the tissue, preferably based on the radiative transfer theory and its approximations, by means of the calculating unit (1144), assuming estimated volume fractions of the individual tissue components which are present in the tissue,
- to adapt the theoretical remission spectrum to the measured remission spectrum by, for example, a non-linear regression, a neural network or a look-up table using the calculating unit (1144), and
- to calculate at least one volume fraction of a tissue component from the remission spectrum via a minimization algorithm with which the theoretically calculated remission spectrum is fitted (adapted) to the measured remission spectrum by means of the calculating unit (1144) by varying the volume fractions of the individual tissue components present in the tissue,
- to calculate at least an absorption maximum from an absorption spectrum of the tissue, wherein the absorption spectrum of the tissue is determined from the fit of the theoretically calculated remission spectrum to the measured remission spectrum,
- to calculate a temperature in the tissue by comparing the absorption maximum with at least one reference,
- to control and/or adjust and/or switch off the medical high frequency surgical instrument (1000, 1100) based on the calculated volume fraction of a tissue component and the calculated temperature, or based on the calculated volume fraction of a tissue component, on the calculated temperature and on the calculated tissue impedance.

2. The medical high frequency surgical instrument (1000, 1100) according to patent claim 1, which is moreover provided and adapted to
- store at least one reference in the form of an absorption maximum at a certain temperature in the calculating unit (1144), preferably a storage medium in the calculating unit (1144), preferably for water and/or fat and/or collagen.

3. The medical high frequency surgical instrument (1000, 1100) according to any one of the preceding patent claims, which is moreover provided, adapted and configured to
- apply at least one light source (10, 110, 210, 310, 410, 510, 610, 710, 810, 910, 1110) and the at least one sensor (12, 112, 212, 312, 412, 512, 612, 712, 812, 912, 1112) to the tissue.

4. The medical high frequency surgical instrument (1000, 1100) according to patent claim 1, **characterized in that** the medical high frequency surgical instrument (1100) is provided, adapted and configured to control and/or to adjust and/or to switch off, when a predetermined temperature is reached, preferably at a temperature larger than 95° Celsius and less than 100° Celsius.

5. The medical high frequency surgical instrument (1000, 1100) according to any one of the preceding patent claims, **characterized in that** the medical high frequency surgical instrument (1000, 1100) is provided, adapted and configured to control and/or adjust and/or to switch off the medical high frequency surgical instrument (1000, 1100) online, preferably in real time.

6. The medical high frequency surgical instrument (1000, 1100) according to any one of the preceding patent claims, **characterized in that** the medical high frequency surgical instrument (1000, 1100) is provided, adapted and configured in order to perform the tissue identification during a sealing process.

7. The medical high frequency surgical instrument (1000, 1100) according to any one of the preceding patent claims, **characterized in that** the at least one sensor (12, 112, 212, 312, 412, 512, 612, 712, 812, 912, 1120) is provided and adapted to measure the remission, preferably the remission spectra, in the NIR range of 1000 nm to 1700 nm, more preferably in the range of 1400 nm to 1600 nm.

8. The medical high frequency surgical instrument (1000, 1100) according to any one of the preceding patent claims, **characterized in that** the at least one light source (10, 110, 210, 310, 410, 510, 610, 710, 810, 910, 1100) and the at least one sensor (12, 112, 212, 312, 412, 512, 612, 712, 812, 912, 1120) are spaced apart from each other.

9. The medical high frequency surgical instrument (1000, 1100) according to any one of the preceding patent claims, **characterized in that** the instrument branch (1, 101, 201, 301, 401, 501, 601, 701, 801, 901) forms an instrument branch body (8, 108, 208, 308, 408, 508, 608, 708, 808, 908, 1128), which forms one half of an operable instrument jaw of the medical high frequency surgical instrument (1000, 1100), and comprises at least one electrode (2, 102, 202, 302, 402, 502, 602, 702, 802, 902), which is provided and adapted to come into contact with the tissue, and is arranged in or on the instrument branch body (8, 108, 208, 308, 408, 508, 608, 708, 808, 908, 1128), wherein
the at least one light source (10, 510, 810,1100) and the at least one sensor (12, 512, 812, 1120) are arranged in or on the instrument branch body (8, 508, 808, 1128).

10. The medical high frequency surgical instrument (1000, 1100) according to any one of the preceding patent claims 1 to 8, **characterized in that** the instrument branch (1, 101, 201, 301, 401, 501, 601, 701, 801, 901, 1001, 1002) forms an instrument branch body (8, 108, 208, 308, 408, 508, 608, 708, 808, 908, 1128), which forms one half of an operable instrument jaw of the medical high frequency surgical instrument (1100), and comprises at least one electrode (2, 102, 202, 302, 402, 502, 602, 702, 802, 902), which is provided and adapted to come into contact with the tissue, and is arranged in or on the instrument branch body (8, 108, 208, 308, 408, 508, 608, 708, 808, 908, 1128), wherein
at least one light tunnel (120, 220, 320, 420, 620, 720, 820, 920) is arranged in or on the instrument branch (101, 201, 301, 401, 501, 601, 701, 801, 901, 1001, 1002), through which the first light from the at least one light source (110, 210, 310, 410, 510, 610, 710, 810, 910) and/or the second light is directed from the tissue.

11. A machine readable storage medium **characterized by** steps stored on the storage medium, which the calculating unit (1114) of the medical high frequency surgical instrument (1000, 1100) executes according to one of the preceding claims 1 to 10.

## Revendications

1. Instrument chirurgical médical à haute fréquence (1000, 1100) avec
- au moins une branche d'instrument (1, 101, 201, 301, 401, 501, 601, 701, 801, 901, 1001, 1002),
- au moins une source de lumière (10, 110, 210, 310, 410, 510, 610, 710, 810, 910, 1110), en particulier une LED ou un module de sources de lumière, en particulier une LED et un filtre, qui génère une première lumière avec un spectre de lumière d'éclairage déterminé, laquelle peut être envoyée dans la direction d'un tissu, et
- au moins un capteur (12, 112, 212, 312, 412, 512, 612, 712, 812, 912, 1112) qui est prévu et adapté pour acquérir une seconde lumière avec préférentiellement un spectre de rémission différent du spectre de lumière d'éclairage, qui est réfléchi par le tissu à la suite de l'exposition à la lumière par le biais de la source de lumière (10, 110, 210, 310, 410, 510, 610, 710, 810, 910, 1110) et
- pour convertir la seconde lumière conformément au spectre de rémission de celle-ci en un signal de détection, dans lequel
une unité de calcul (1144) est prévue et réalisée pour
- recevoir le signal de détecteur de l'au moins un capteur (12, 112, 212, 312, 412, 512, 612, 712, 812, 912, 1112),
- calculer un spectre de rémission théorique sur la base d'une solution destinée à la description du rayonnement de lumière dans le tissu, de préférence sur la base de la théorie de transport de rayonnement et de ses approximations, au moyen de l'unité de calcul (1144), en supposant des parts de volume estimées des différents composants tissulaires qui sont présents dans le tissu,
- adapter le spectre de rémission théorique au spectre de rémission mesuré par exemple par le biais d'une régression non linéaire, d'un réseau neuronal ou d'une table de correspondance au moyen de l'unité de calcul (1144), et
- calculer au moins une part de volume d'un composant tissulaire à partir du spectre de rémission par le biais d'un algorithme de minimisation avec lequel le spectre de rémission calculé de manière théorique est ajusté (adapté) au spectre de rémission mesuré au moyen de l'unité de calcul (1144) par le biais d'une variation des parts de volume des différents composants tissulaires qui sont présents dans le tissu,
- calculer au moins un maximum d'absorption à partir d'un spectre d'absorption du tissu, dans lequel le spectre d'absorption du tissu est déterminé à partir de l'ajustement du spectre de rémission calculé de manière théorique au spectre de rémission mesuré,
- calculer une température dans le tissu par le biais d'une comparaison du maximum d'absorption à au moins une référence,
- commander et/ou réguler et/ou couper l'alimentation de l'instrument chirurgical médical à haute fréquence (1000, 1100) sur la base de la part de volume calculée d'un composant tissulaire et de la température calculée, ou sur la base de la part de volume calculée d'un composant tissulaire, de la température calculée et de l'impédance tissulaire calculée.

2. Instrument chirurgical médical à haute fréquence (1000, 1100) selon la revendication 1, qui est en outre prévu, adapté et réalisé pour
- enregistrer au moins une référence sous la forme d'un maximum d'absorption à une température déterminée dans l'unité de calcul (1144), de préférence un support d'enregistrement dans l'unité de calcul (1144), de préférence pour de l'eau et/ou de la graisse et/ou du collagène.

3. Instrument chirurgical médical à haute fréquence (1000, 1100) selon l'une quelconque des revendications précédentes, qui est en outre prévu, adapté et réalisé pour
- appliquer l'au moins une source de lumière (10, 110, 210, 310, 410, 510, 610, 710, 810, 910, 1110) et l'au moins un capteur (12, 112, 212, 312, 412, 512, 612, 712, 812, 912, 1112) sur le tissu.

4. Instrument chirurgical médical à haute fréquence (1000, 1100) selon la revendication 1, **caractérisé en ce que** l'instrument chirurgical médical à haute fréquence (1100) est prévu, adapté et réalisé pour commander et/ou réguler et/ou couper l'alimentation lorsqu'une température prédéterminée est atteinte, de préférence à une température qui est supérieure à 95 ° Celsius et inférieure à 100 ° Celsius.

5. Instrument chirurgical médical à haute fréquence (1000, 1100) selon l'une quelconque des revendications précédentes, **caractérisé en ce que** l'instrument chirurgical médical à haute fréquence (1000, 1100) est prévu, adapté et réalisé pour commander et/ou réguler et/ou couper l'alimentation en ligne de l'instrument chirurgical médical à haute fréquence (1000, 1100), de préférence en temps réel.

6. Instrument chirurgical médical à haute fréquence (1000, 1100) selon l'une quelconque des revendications précédentes, **caractérisé en ce que** l'instrument chirurgial médical à haute fréquence (1000, 1100) est prévu, adapté et réalisé pour exécuter la reconnaissance tissulaire pendant un processus d'étanchéification.

7. Instrument chirurgical médical à haute fréquence (1000, 1100) selon l'une quelconque des revendications précédentes, **caractérisé en ce que** l'au moins un capteur (12, 112, 212, 312, 412, 512, 612, 712, 812, 912, 1120) est prévu et adapté pour mesurer la rémission, de préférence les spectres de rémission, dans la plage PIR de 1000 nm à 1700 nm, le plus préférentiellement dans la plage de 1400 nm à 1600 nm.

8. Instrument chirurgical médical à haute fréquence (1000, 1100) selon l'une quelconque des revendications précédentes, **caractérisé en ce que** l'au moins une source de lumière (10, 110, 210, 310, 410, 510, 610, 710, 810, 910, 1100) et l'au moins un capteur (12, 112, 212, 312, 412, 512, 612, 712, 812, 912, 1120) sont espacés.

9. Instrument chirurgical médical à haute fréquence (1000, 1100) selon l'une quelconque des revendications précédentes, **caractérisé en ce que** la branche d'instrument (1, 101,201,301,401,501, 601,701, 801,901) présente un corps de branche d'instrument (8, 108, 208, 308, 408, 508, 608, 708, 808, 908, 1128) qui réalise une moitié d'un embout d'instrument actionnable de l'instrument chirurgical médical à haute fréquence (1000, 1100) et au moins une électrode (2, 102, 202, 302, 402, 502, 602, 702, 802, 902) qui est prévue et adaptée pour venir en contact avec le tissu et est disposée dans ou sur le corps de branche d'instrument (8, 108, 208, 308, 408, 508, 608, 708, 808, 908, 1128), dans lequel
l'au moins une source de lumière (10, 510, 810, 1100) et l'au moins un capteur (12, 512, 812, 1120) sont disposés dans ou sur le corps de branche d'instrument (8, 508, 808, 1128).

10. Instrument chirurgical médical à haute fréquence (1000, 1100) selon l'une quelconque des revendications 1 à 8, **caractérisé en ce que** la branche d'instrument (1, 101, 201, 301, 401, 501, 601, 701, 801,901, 1001, 1002) présente un corps de branche d'instrument (8, 108, 208, 308, 408, 508, 608, 708, 808, 908, 1128) qui réalise une moitié d'un embout d'instrument actionnable de l'instrument chirurgical médical à haute fréquence (1000, 1100) et au moins une électrode (2, 102, 202, 302, 402, 502, 602, 702, 802, 902) qui est prévue et adaptée pour venir en contact avec le tissu et est disposée dans ou sur le corps de branche d'instrument (8, 108, 208, 308, 408, 508, 608, 708, 808, 908, 1128), dans lequel
au moins un tunnel de lumière (120, 220, 320, 420, 620, 720, 820, 920) est disposé dans ou sur la branche d'instrument (101,201, 301,401, 501,601,701,801, 901, 1001, 1002) à travers lequel la première lumière est dirigée par l'au moins une source de lumière (110, 210, 310, 410, 510, 610, 710, 810, 910) et/ou la seconde lumière à partir du tissu.

11. Support d'enregistrement lisible par machine **caractérisé par** des étapes enregistrées sur le support d'enregistrement que réalise l'unité de calcul (1114) de l'instrument chirurgical médical à haute fréquence (1000, 1100) selon l'une quelconque des revendications précédentes 1 à 10.
